(19) Europäisches Patentamt
European Patent Office
Office européen des brevets

(11) EP 0 832 104 B1

(12) **EUROPEAN PATENT SPECIFICATION**

(45) Date of publication and mention of the grant of the patent:
**04.09.2002 Bulletin 2002/36**

(21) Application number: **96918660.0**

(22) Date of filing: **03.06.1996**

(51) Int Cl.[7]: **C07K 7/06**, A61K 38/08

(86) International application number:
**PCT/EP96/02392**

(87) International publication number:
**WO 96/040751 (19.12.1996 Gazette 1996/55)**

(54) **DOLASTATIN DERIVATIVES, THEIR PREPARATION AND USE**

DERIVATE VON DOLASTATIN, DREEN HERSTELLUNG UN VERWENDUNG

DERIVES DE LA DOLASTATINE, LEURS PREPARATION ET LEURS UTILISATION

(84) Designated Contracting States:
**AT BE CH DE DK ES FI FR GB GR IE IT LI LU MC NL PT SE**
Designated Extension States:
**SI**

(30) Priority: **07.06.1995 US 472453**

(43) Date of publication of application:
**01.04.1998 Bulletin 1998/14**

(73) Proprietor: **Abbott GmbH & Co. KG**
**65205 Wiesbaden (DE)**

(72) Inventors:
- **HAUPT, Andreas**
  **Westborough, MA 01581 (US)**
- **EMLING, Franz**
  **D-67065 Ludwigshafen (DE)**
- **ROMERDAHL, Cynthia, A.**
  **Wayland, MA 01778 (US)**

(74) Representative: **Riedl, Peter, Dr. et al**
**Patentanwälte**
**Reitstötter, Kinzebach & Partner**
**Postfach 86 06 49**
**81633 München (DE)**

(56) References cited:
**WO-A-93/23424**

- **TETRAHEDRON, vol. 50, no. 42, 17 October 1994, OXFORD GB, pages 12097-12108, XP002015211 G R PETTITT ET AL.: "The dolastatins 20. A convenient synthetic route to Dolastatin 15"**

Remarks:
The file contains technical information submitted after the application was filed and not included in this specification

EP 0 832 104 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to dolastatin derivatives, their preparation and use as well as to pharmaceutical compositions containing said derivatives.

It is known that peptides isolated from marine origin like Dolastatin-10 (US 4,816,444) and Dolastatin-15 (EP-A-398558) show potent cell growth inhibitory activity (cf.: Biochem. Pharmacology 40, no. 8, 1859-64, 1990); J. Natl. Cancer Inst. 85, 483-88, 1993 and references cited therein). Based upon interesting results in experimental tumor systems in vivo, further preclinical evaluation of these natural products is currently under way in order to initiate clinical studies in cancer patients. However, the natural products have disadvantages, such as poor solubility in aqueous solvents and costly building blocks needed for synthesis.

WO 93/23424 describes derivatives of dolastatin having the formula

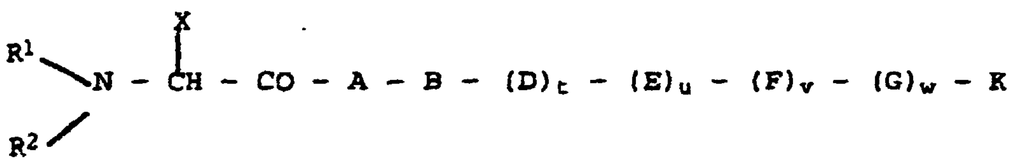

I

where

$R^1$ is alkoxy; alkyl; cycloalkyl; alkylsulfonyl; fluoroalkyl; trifluoroacetyl; amidino; ureyl; piperidinosulfonyl; morpholinosulfonyl; benzyloxycarbonyl; alkyloxycarbonyl; aminosulfonyl which may be substituted by alkyl; hydroxy; arylsulfonyl which may be substituted by one or more substituents independently selected from alkyl, $-N(CH_3)_2$, nitro, halogen and $CF_3$; benzyl which may be substituted by up to three substituents independently selected from alkyl, alkoxy, nitro, halogen and $CF_3$; or $NR^3R^4$ where $R^3$ and $R^4$ may each be either hydrogen or alkyl;

R2 is hydrogen; alkyl; fluoroalkyl; cycloalkyl; acyl; benzoyl or benzyl which may both be substituted by up to three substituents independently selected from nitro, halogen, $CF_3$, alkyl and alkoxy

$R^1$-N-$R^2$ together may be phthalimido, a 5- or 6-membered heterocycle which may be unsubstituted or substituted with one or more substituents independently selected from phenyl, benzyl, alkyl, $N(CH_3)_2$, nitro, thienyl, $CONH_2$ and COOEt

A is a valyl, isoleucyl, leucyl, allo-isoleucyl, $\alpha$-aminoisobutanoyl, 3-tert-butylalanyl, 2-tert-butylglycyl, 3-cyclohexylalanyl, 2,4-diaminobutanoyl, ornithyl, lysyl, 2-ethylglycyl, 2-cyclohexylglycyl, norleucyl, norvalyl or arginyl residue;

B is a N-alkyl-valyl, -norvalyl, -leucyl, -isoleucyl, -2-tert-butylglycyl, -3-tert-butylalanyl, -3-cyclohexylalanyl, -phenylalanyl, or -2-cyclohexylglycyl residue;

D,E,F and G are independently selected from the group consisting of prolyl, homo-prolyl, hydroxyprolyl, thiazolidinyl-4-carbonyl, 1-aminopentyl-1-carbonyl, valyl, 2-tert-butylglycyl, isoleucyl, leucyl, 3-cyclohexylalanyl, phenylalanyl, N-methylphenylalanyl, tetrahydroisoquinolyl-2-carbonyl, 3-thiazolylalanyl, 3-thienylalanyl, histidyl, 1-aminoindyl-1-carbonyl, 2,4-diaminobutanoyl, arginyl, 3-pyridylalanyl, 3-tert-butylalanyl, 2-cyclohexylglycyl, lysyl, norvalyl, norleucyl and 3-naphthylalanyl residues

x is hydrogen, alkyl, cycloalkyl, $-CH_2$-cyclohexyl or arylalkyl

A and B together, F and G together, $R^1R^2$N-CHX-CO and A together, E and F together, either alone or in pairs, may be

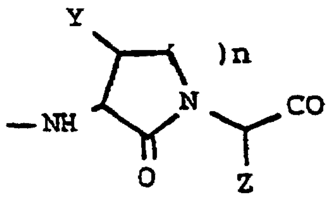

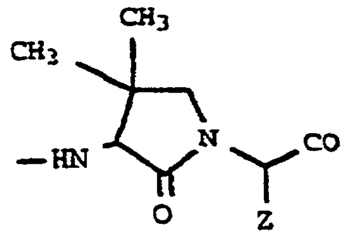

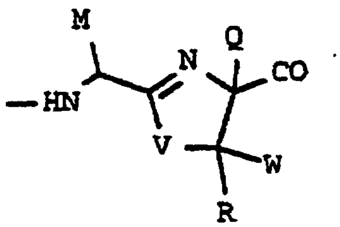

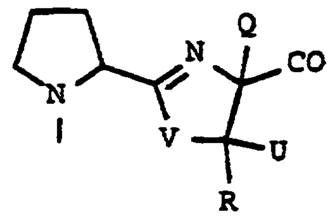

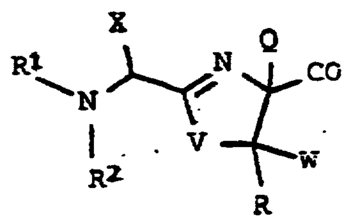

where Y is hydrogen or lower alkyl; Z is hydrogen or lower alkyl; n is 1, 2, or 3; V is oxygen or sulfur; M is hydrogen, lower alkyl, arylalkyl, cyclohexyl, or -$CH_2$-cyclohexyl; Q is hydrogen; R is hydrogen or lower alkyl; or R and Q may together form a bond; U is hydrogen, lower alkyl, phenyl, or cycloalkyl; and W is hydrogen, lower alkyl or phenyl;

t,u,v,and w are independently 0 or 1; and

K is hydroxy, alkoxy, phenoxy, benzyloxy or a substituted or unsubstituted amino moiety;

provided that where t, u, v and w are 0, K is not a hydroxy, alkoxy, benzoxy or phenoxy moiety; and further provided that where t, u and v are 0, K is not a hydroxy or alkoxy moiety;
and the salts thereof with physiologically tolerated acids.

The invention described herein provides novel peptides and derivatives thereof which offer improved therapeutic potential for the treatment of neoplastic diseases as compared to Dolastatin-15. Furthermore, the compounds of this invention may be conveniently synthesized as described in detail below.

Compounds of this invention include novel peptides of the formula I

$$R^1R^2N\text{-}CHX\text{-}CO\text{-}A\text{-}B\text{-}D\text{-}E\text{-}(F)_t\text{-}K \qquad I$$

where

$R^1$ is methyl, ethyl, or isopropyl;

$R^2$ is hydrogen, methyl, or ethyl;

$R^1$-N-$R^2$ together may be a pyrrollidine ring;

A is a valyl, isoleucyl, leucyl, 2-tert-butylglycyl, 2-ethylglycyl, norleucyl or norvalyl residue;

B is a N-methyl-valyl, -leucyl, -isoleucyl, -norvalyl, -norleucyl -2-tert-butylglycyl, -3-tert-butylalanyl, or -2-ethylglycyl residue;

D is a 3,4-dehydroprolyl, 4-fluoroprolyl, 4,4-difluoroprolyl, azetidine-2-carbonyl, 3-methylprolyl, 4-methylprolyl, or 5-methylprolyl residue;

E is a prolyl, homoprolyl, hydroxyprolyl or thiazolidine-4-carbonyl residue;

F is a valyl, 2-tert-butylglycyl, isoleucyl, leucyl, 2-cyclohexylglycyl, norleucyl, norvalyl, neopentylglycyl, alanyl, β-alanyl, or aminoisobutyroyl residue;

x is alkyl (preferably $C_{2-5}$), cyclopropyl, or cyclopentyl;

t is 0 or 1; and

K is $C_{1-4}$-alkoxy, benzyloxy, $-NH_2$, -NH-$C_{1-12}$-alkyl, $-NH-C(CH_3)_2CN$, $-NH-C(CH_3)_2CCH$, $-NH-C(CH_3)_2C=CH_2$, $-NH-C(CH_3)_2CH_2CH_2OH$, $-NH-C(CH_3)_2CH_2OH$, -NH-$C_{3-8}$-cycloalkyl, -NH-[3,3,0]-bicyclooctyl, norephedryl, norpseudoephedryl, -NH-quinolyl, -NH-pyrazyl, -NH-$CH_2$-benzimidazolyl, -NH-adamantyl, -NH-$CH_2$-adamantyl, -NH-CH($CH_3$)-phenyl, -NH-C$(CH_3)_2$-phenyl, -N($C_{1-4}$-alkoxy)-$C_{1-4}$-alkyl, -N($C_{1-4}$-alkoxy)-$CH_2$-phenyl, -N($C_{1-4}$-alkoxy)-phenyl, -N($CH_3$)OBzl, -NH-$(CH_2)_v$-phenyl (v=0,1,2,or 3), -NH-$(CR_2)_m$-naphthyl (m=0 or 1), -NH-$(CH_2)_w$-benzhydryl (w=0,1, or 2), -NH-biphenyl, -NH-pyridyl, -NH-$CH_2$-pyridyl, -NH-$CH_2$-$CH_2$-pyridyl, -NH-benzothiazolyl, -NH-benzoisothiazolyl, -NH-benzopyrazolyl, -NH-benzoxazolyl, -NH-$(CH_2)_m$-fluorenyl (m=0 or 1), -NH-pyrimidyl, -NH-$(CH_2)_m$-indanyl (m=0 or 1), $-NH-(CH_2CH_2O)_y-CH_3$ (y=0,1,2,3,4, or 5), $-NH-(CH_2CH_2O)_y-CH_2CH_3$ (y=0,1,2,3,4, or 5), $-(CH_2CH_2O)_y-CH_3$ (y=0,1,2,3,4, or 5), $-(CH_2CH_2O)_y-CH_2CH_3$ (y=0,1,2,3,4, or 5), $-NCH_3-NH-C_6H_5$, $-NCH_3-NH-CH_2-C_6H_5$; or K is

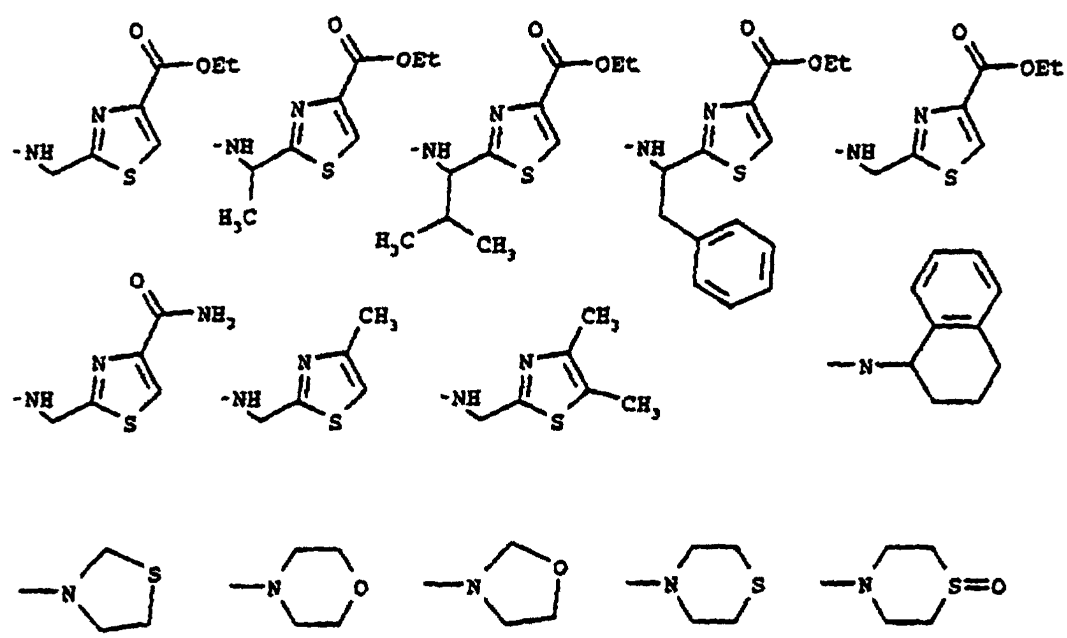

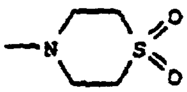
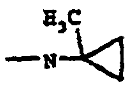
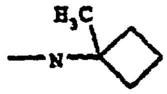
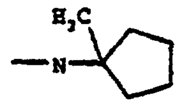

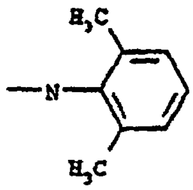
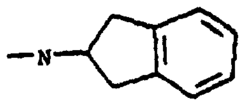
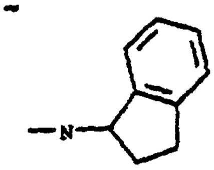
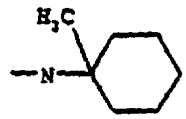

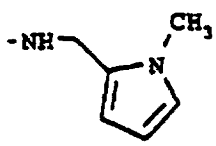
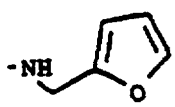
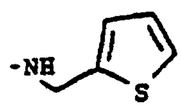
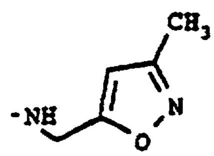
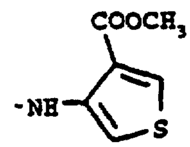

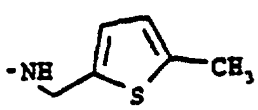
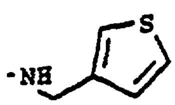
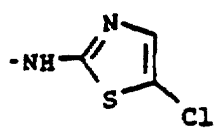
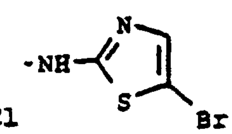
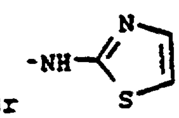

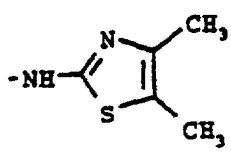
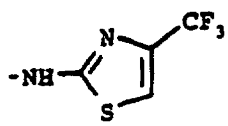
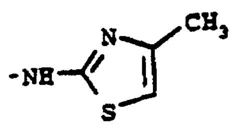
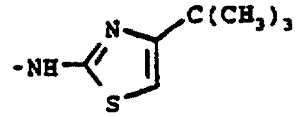

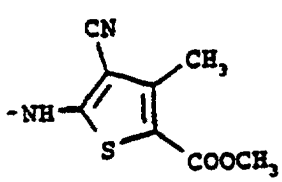
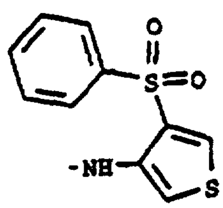
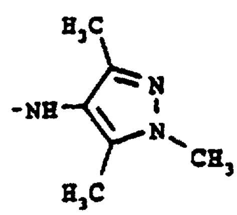
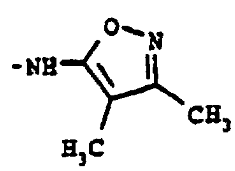

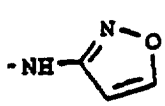
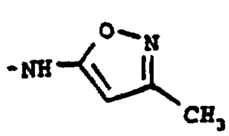
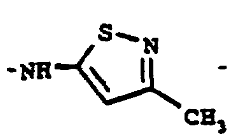
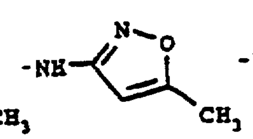
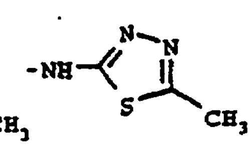

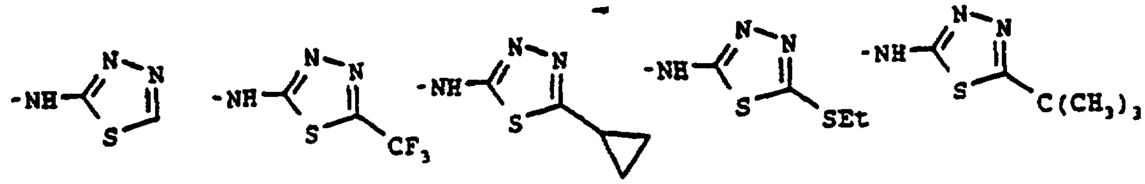

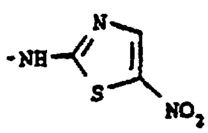
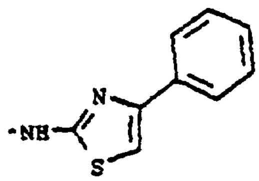
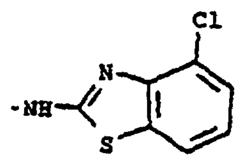
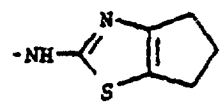

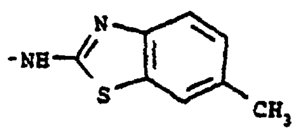
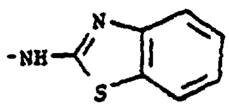
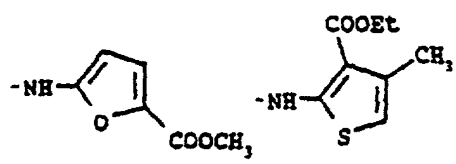

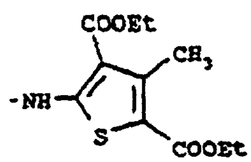
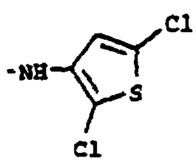
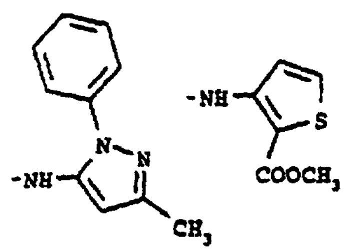
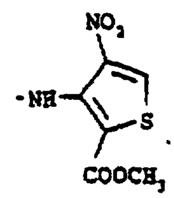

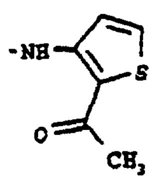
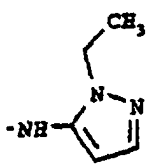
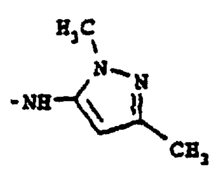
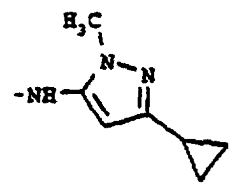

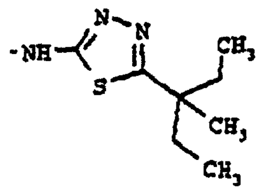
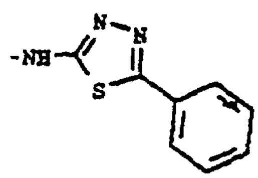
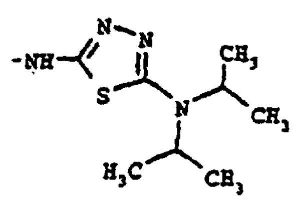

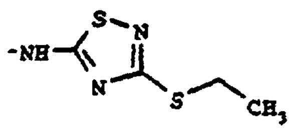
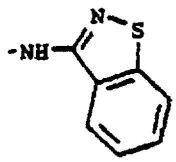
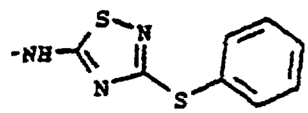

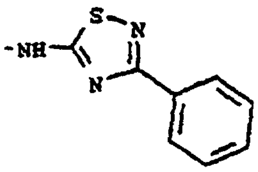
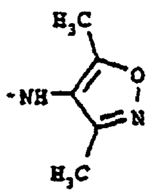
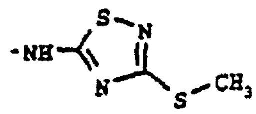

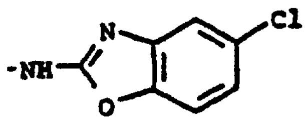
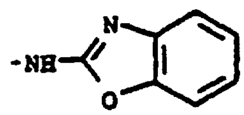
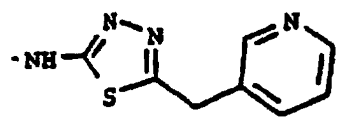

Compounds of this invention also include the salts of the peptides with physiologically tolerated acids.

This invention also provides methods for preparing the compounds of formula I, pharmaceutical compositions containing such compounds together with a pharmaceutically acceptable carrier and methods for using same for treating cancer in mammals.

Preferred are compounds of the formula I where the substituents $R^1$, $R^2$, A, B, D, E, X, F and t have the following meanings

$R^1$ is methyl or ethyl;

$R^2$ is hydrogen, methyl, or ethyl;

A is a valyl, isoleucyl, 2-tert-butylglycyl residue;

B is a N-methyl-valyl, -isoleacyl, or -2-tert-butylglycyl residue;

D is a 3,4-dehydroprolyl, 4-fluoroprolyl, azetidine-2-carbonyl, 3-methylprolyl, or 5-methyl-prolyl residue;

E is a prolyl, homoprolyl, hydroxyprolyl or thiazolidine-4-carbonyl residue;

F is a D-valyl, 2-tert-butylglycyl, D-isoleucyl, D-leucyl, or aminoisobutyroyl residue;

X is $-CH(CH_3)_2$, $-C(CH_3)_3$, or $-CH(CH_3)CH_2CH_3$;

t is 0 or 1.

Preferred meanings for K are:
$-OC(CH_3)_3$, $-NHCH_3$, $-NHCH_2CH_3$, $-NH(CH_2)_2CH_3$, $-NH(CH_2)_3CH_3$, $-NH(CH_2)_4CH_3$, $-NH(CH_2)_5CH_3$, $-NH(CH_2)_6CH_3$, $-NH(CH_2)_7CH_3$, $-NHCH(CH_3)_2$, $-NHCH(CH_3)CH_2CH_3$, $-NHCH(CH_3)CH_2CH_2CH_3$, $-NHCH(CH_2CH_3)_2$, $-NH(CH_2CH_2CH_3)_2$, $-NHC(CH_3)_3$, $-NHCH(CH_2CH_3)CH_2CH_2CH_3$, $-NHCH(CH_3)CH(CH_3)_2$, $-NHCH(CH_2CH_3)CH(CH_3)_2$, $-NHCH(CH_3)C(CH_3)_3$, -NH-cyclopropyl, -NH-cyclobutyl, -NH-cyclopentyl, -NH-cyclohexyl, -NH-cycloheptyl, -NH-cyclooctyl, -NH-bicycle[3,3,0]octyl, $-N(CH_3)OCH_3$, $-N(CH_3)OCH_2CH_3$, $-N(CH_3)OCH_2CH_2CH_3$, $-N(CH_3)OCH(CH_3)_2$, $-N(CH_2CH_3)OCH_3$, $-N(CH_2CH_3)OCH_2CH_3$, $-N(CH(CH_3)_2)OCH_3$, $-N(CH_3)OCH_2C_6H_5$, $-N(OCH_3)CH_2-C_6H_5$, $-N(CH_3)OC_6H_5$, $-NH-CH_2-C_6H_5$, $-NH(CH_2)_2C_6H_5$, $-NH(CH_2)_3C_6H_5$, $-NHCH(CH_3)C_6H_5$, $-NHC(CH_3)_2C_6H_5$, -NHC

$(CH_3)_2CH_2CH_3$, $-NHC(CH_3)(CH_2CH_3)_2$, $-NHCH(CH_3)CH(OH)C_6H_5$, $-NHCH_2$-cyclohexyl, $-NH-CH_2CF_3$, $-NHCH(CH_2F)_2$, $-NHC(CH_3)_2CH_2CH_2OH$, $-NH(CH_2CH_2O)_2CH_2CH_3$, $-NHC(CH_3)_2CH(CH_3)_2$, $-NHC(CH_3)_2CN$, $-NHC(CH_3)_2CCH$, norephedryl, norpseudoephedryl, -NH-quinolyl, -NH-pyrazyl, -NH-adamantyl(2), -NH-adamantyl(1), $-NH-CH_2$-adamantyl, $-NH-CH_2$-naphthyl, -NH-benzhydryl, -NH-biphenyl, -NH-pyridyl, $-MH-CH_2$-pyridyl, $-NH-CH_2-CH_2$-pyridyl, -NH-benzothiazolyl, -NH-benzoisothiazolyl, -NH-benzopyrazolyl, -NH-benzoxazolyl, -NH-fluorenyl, -NH-pyrimidyl, $-NH-CH_2$-(4-methyl)-thiazolyl(2), $-NH-CH_2$-furanyl(2), $-NH-CH_2$-thienyl(2), $-NH-CH_2$-(5-methyl)thienyl(2), -NH-thiazolyl(2), -NH-isoxazolyl(3), -NH-(3-methyl)isoxazolyl(5), -NH-(3-methyl)isothiazolyl(5), -NH-(2-trifluormethyl)-thiadiazolyl(5), -NH-(2-cyclopropyl)thiadiazolyl(5), $-NHC(CH_3)_2C=CH_2$, or K may be

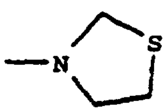
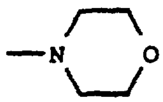
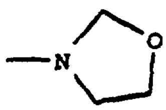
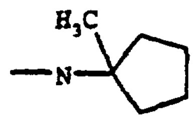
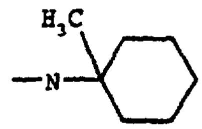

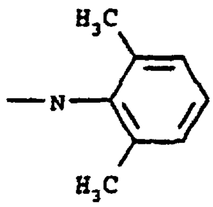
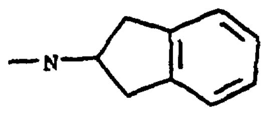
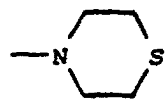
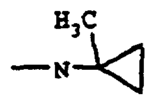
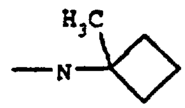

These examples illustrate but do not limit the scope of the present invention.

The peptides of the formula I are composed of L-amino acids but F may also be a D-amino acid.

The new compounds may be present as salts with physiologically tolerated acids such as: hydrochloric acid, citric acid, tartaric acid, lactic acid, phosphoric acid, methanesulfonic acid, acetic acid, formic acid, maleic acid, fumaric acid, malic acid, succinic acid, malonic acid, sulfuric acid, L-glutamic acid, L-aspartic acid, pyruvic acid, mucic acid, benzoic acid, glucuronic acid, oxalic acid, ascorbic acid and acetylglycine.

The novel compounds can be prepared by known methods of peptide chemistry. Thus, the peptides can be assembled sequentially from amino acids or by linking suitable small peptide fragments. In the sequential assemblage, starting at the C terminus the peptide chain is extended stepwise by one amino acid each time. In fragment coupling it is possible to link together fragments of different lengths, and the fragments in turn can be obtained by sequential assemblage from amino acids or themselves by fragment coupling.

Both in the sequential assemblage and in the fragment coupling it is necessary to link the units by forming an amide linkage. Enzymatic and chemical methods are suitable for this.

Chemical methods for forming the amide linkage are described in detail by Mueller, Methoden der organischen Chemie Vol. XV/2, pp 1 to 364, Thieme Verlag, Stuttgart, 1974; Stewart, Young, Solid Phase Peptide Synthesis, pp 31 to 34, 71 to 82, Pierce Chemical Company, Rockford, 1984; Bodanszky, Klausner, Ondetti, Peptide Synthesis, pp 85 to 128, John Wiley & Sons, New York, 1976 and other standard works on peptide chemistry. Particular preference is given to the azide method, the symmetric and mixed anhydride method, in situ generated or preformed active esters, the use of urethane protected N-carboxy anhydrides of amino acids and the formation of the amide linkage using coupling reagents/activators, especially dicyclohexylcarbodiimide (DCC), diisopropylcarbodiimide (DIC ), 1-ethoxycarbonyl-2-ethoxy-1,2-dihydroquinoline (EEDQ), 1-ethyl-3-(3-dimethylaminopropyl)carbodiimide hydrochloride (EDCI), n-propanephosphonic anhydride (PPA), N,N-bis(2-oxo-3-oxazolldinyl)-amidophosphoryl chloride (BOP-Cl), bromo-tris-pyrrolidinophosphonium hexafluorophosphate (PyBrop), diphenylphosphoryl azide (DPPA), Castro's reagent (BOP, PyBop), o-benzotriazolyl-N,N,N',N'-tetramethyluronium salts (HBTU), O-Azabenzotriazolyl-N,N,N',N'-tetramethyluronium salts (HATU), diethylphosphoryl cyanide (DEPCN), 2,5-diphenyl-2,3-dihydro-3-oxo-4-hydroxythiophene dioxide (Steglich's reagent; HOTDO) and 1,1'-carbonyldiimidazole (CDI). The coupling reagents can be employed alone or in combination with additives such as N,N-dimethyl-4-aminopyridine (DMAP), N-hydroxy-benzotriazole (HOBt), N-hydroxybenzotriazine (HOOBt), Azabenzotriazole (HOAt), N-hydroxysuccinimide (HOSu) or 2-hydroxypyridine.

Whereas it is normally possible to dispense with protective groups in enzymatic peptide synthesis, reversible protection of reactive groups not involved in formation of the amide linkage is necessary for both reactants in chemical synthesis. Three conventional protective group techniques are preferred for the chemical peptide synthesis: the benzyloxycarbonyl (Z), the t-butoxycarbonyl (Boc) and the 9-fluorenylmethoxycarbonyl (Fmoc) techniques. Identified in each case is

the protective group on the alpha-amino group of the chain-extending unit. A detailed review of amino-acid protective groups is given by Mueller, Methoden der organischen Chemie Vol. XV/1, pp 20 to 906, Thieme Verlag, Stuttgart, 1974. The units employed for assembling the peptide chain can be reacted in solution, in suspension or by a method similar to that described by Merrifield in J. Amer. Chem. Soc. 85 (1963) 2149. Particularly preferred methods are those in which peptides are assembled sequentially or by fragment coupling using the Z, Boc or Fmoc protective group technique.

One of the reactants in the said Merrifield technique is being bonded to an insoluble polymeric support (also called resin hereinafter). This typically entails the peptide being assembled sequentially on the polymeric support using the Boc or Fmoc protective group technique, the growing peptide chain being covalently bonded at the C terminus to the insoluble resin particles (cf. Fig. 1 and 2). This procedure makes it possible to remove reagents and byproducts by filtration, and thus recrystallization of intermediates is unnecessary.

The protected amino acids can be linked to any suitable polymers, which merely have to be insoluble in the solvents used and to have a stable physical form which makes filtration easy. The polymer must contain a functional group to which the first protected amino acid can be firmly attached by a covalent bond. Suitable for this purpose are a wide variety of polymers, eg. cellulose, polyvinyl alcohol, polymethacrylate, sulfonated polystyrene, chloromethylated styrene/divinylbenzene copolymer (Merrifield resin), 4-methylbenzhydrylamine resin (MBHA-resin), phenylacetamidomethyl-resin (Pam-resin), p-benzyloxy-benzyl-alcohol-resin, benzhydryl-amine-resin (BHA-resin), 4-(hydroxymethyl)-benzoyloxy-methyl-resin, the resin of Breipohl et al. (Tetrahedron Letters 28 (1987) 565; supplied by BACHEM), 4-(2,4-dimethoxyphenylaminomethyl)phenoxy-resin (supplied by Novabiochem) or o-chlorotrityl-resin (supplied by Biohellas).

Suitable for peptide synthesis in solution are all solvents which are inert under the reaction conditions, especially water, N,N-dimethylformamide (DMF), dimethyl sulfoxide (DMSO), acetonitrile, dichloromethane (DCM), 1,4-dioxane, tetrahydrofuran (THF), N-methyl-2-pyrrolidone (NMP) and mixtures of the said solvents. Peptide synthesis on the polymeric support can be carried out in all inert organic solvents in which the amino-acid derivatives used are soluble. However, preferred solvents additionally have resin-swelling properties, such as DMF, DCM, NMP, acetonitrile and DMSO, and mixtures of these solvents. After synthesis is complete, the peptide is cleaved off the polymeric support. The conditions under which cleavage off the various resin types is possible are disclosed in the literature. The cleavage reactions most commonly used are acid- and palladium-catalyzed, especially cleavage in liquid anhydrous hydrogen fluoride, in anhydrous trifluoromethanesulfonic acid, in dilute or concentrated trifluoroacetic acid, palladium-catalyzed cleavage in THF or THF-DCM mixtures in the presence of a weak base such as morpholine or cleavage in acetic acid-dichloromethanetrifluoroethanol mixtures. Depending on the chosen protective groups, these may be retained or likewise cleaved off under the cleavage conditions.

Partial deprotection of the peptide may also be worthwhile when certain derivatization reactions are to be carried out. Peptides dialkylated at the N-terminus can be prepared a) by coupling of the appropriate N,N-di-alkylamino acids in solution or on the polymeric support, b) by reductive alkylation of the resin-bound peptide in DMF/1% acetic acid with $NaCNBH_3$ and the appropriate aldehyde or ketone, c) by hydrogenation of the peptides in solution in the presence of aldehyde or ketone and Pd/C.

The various non-naturally occurring amino acids disclosed herein may be obtained from commercial sources or synthesized from commercially-available materials using methods known in the art. Azetidine-2-carboxylic acid, 3-methyl-L-proline, 5-methyl-L-proline, and Boc- or Fmoc-protected 3,4-dehydroproline are commercially available starting materials (ACROS, NOVABIOCHEM, BACHEM). Cis- and trans-4-fluoroproline can be prepared via a method described by Panasik et al. (N. Panasik, E.S. Eberhardt, A.S. Edison, D.R. Powell, R.T. Raines, Int. J. Peptide Protein Res. 44, 1994, 262-269) from hydroxyproline.

The compounds of this invention may be used to inhibit or otherwise treat solid tumors (e.g. tumors of the lung, breast, colon, prostate, bladder, rectum, or endometrial tumors) or hematological malignancies (e.g. leukemias, lymphomas) by administration of the compound to the mammal.

It is a special advantage of the new compounds that they are more resistant to enzymatic degredation as compared to Dolastatin-15.

Administration may be by any of the means which are conventional for pharmaceutical, preferably oncological, agents, including oral and parenteral means such as subcutaneously, intravenously, intramuscularly and intraperitoneally.

The compounds may be administered alone or in the form of pharmaceutical compositions containing a compound of formula I together with a pharmaceutically accepted carrier appropriate for the desired route of administration. Such pharmaceutical compositions may be combination products, i.e., may also contain other therapeutically active ingredients.

The dosage to be administered to the mammal will contain an effective tumor-inhibiting amount of active ingredient which will depend upon conventional factors including the biological activity of the particular compound employed; the means of administration; the age, health and body weight of the recipient; the nature and extent of the

symptoms; the frequency of treatment; the administration of other therapies; and the effect desired. A typical daily dose will be about 0.5 to 50 milligrams per kilogram of body weight on oral administration and about 0.05 to 20 on parenteral administration.

The novel compounds can be administered in conventional solid or liquid pharmaceutical administration forms, e.g. uncoated or (film-)coated tablets, capsules, powders, granules, suppositories or solutions. These are produced in a conventional manner. The active substances can for this purpose be processed with conventional pharmaceutical aids such as tablet binders, fillers, preservatives, tablet disintegrants, flow regulators, plasticizers, wetting agents, dispersants, emulsifiers, solvents, sustained release compositions, antioxidants and/or propellant gases (cf. H. Sucker et al.: Pharmazeutische Technologie, Thieme-Verlag, Stuttgart, 1978). The administration forms obtained in this way normally contain 1-90% by weight of the active substance.

The following examples are intended to illustrate the invention. The proteinogenous amino acids are abbreviated in the examples using the known three-letter code. Other abbreviations used: $Me_2$Val=N,N-dimethylvaline, MeVal=N-methylvaline, Z=benzyloxycarbonyl.

A. General procedures

I. The peptides claimed in claim 1 are either synthesized by classical solution synthesis using standard Z- and Boc-methodology as described above or by standard methods of solid-phase synthesis using Boc and Fmoc protective group techniques.

a) Synthetic cycle for the Fmoc protective group technique

| | | |
|---|---|---|
| 1. | DMF washing | 1 x 1 min |
| 2. | 20% piperidine in DMF | 1 x 4 min |
| 3. | 20% piperidine in DMF | 1 x 16 min |
| 4. | DMF washing | 5 x 1 min |
| 5. | Addition of the preactivated protected amino acid (activation by 1 equivalent of TBTU and 5 equivalents of DIPEA in DMF); Peptide coupling | 1 x 61 min |
| 6. | DMF washing | 3 x 1 min |
| 7. | if conversion is incomplete, repetition of coupling (back to 5.) | |
| 8. | DMF washing | 3 x 1 min |
| 9. | back to 2. | |

BOP-Cl and PyBrop were used as reagents for coupling of the amino acid following the N-methylamino acids. The reaction times were correspondingly increased including double couplings. In solution synthesis, the use of either Boc-protected amino acid NCA's (N-carboxy anhydrides), Z-protected amino acid NCA's (N-carboxy anhydrides), or the use of pivaloylchloride as condensing agent respectively is most advantageous for this type of coupling.

II. Reductive alkylation of the N-terminus

The peptide-resin prepared as in AIa was deprotected at the N-terminus (steps 2-4 in AIa) and then reacted with a 3-fold molar excess of aldehyde or ketone in DMF/1% acetic acid with addition of 3 equivalents of $NaCNBH_3$. After reaction was complete (negative Kaiser test) the resin was washed several times with water, isopropanol, DMF and dichloromethane.

Reductive alkylation in solution can e.g. be achieved by reaction of the N-terminally deprotected peptides, peptide fragments, or amino acids with the corresponding aldehydes or ketones using $NaCNBH_3$ or hydrogen-Pd/C.

III. Work-up of the peptide-resins obtained as in Ib and II

The peptide-resin was dried under reduced pressure and then subjected to treatment with a TFA/water mixture (95:5) for 1,5 hours (Wade, Tregear, Howard Florey Fmoc Workshop Manual, Melbourne 1985). The resin was then filtered off and washed with TFA and DCM. The filtrate and the washings were concentrated, and the peptide was precipitated by addition of diethyl ether. After cooling in an ice bath, the precipitate was filtered off, taken up in 30% acetic acid and lyophilized.

IV. When an o-chlorotrityl-resin (supplied by Biohellas) is used, the suspension of the peptide-resin in an acetic acid/trifluoroethanol/dichloromethane mixture (1:1:3) is stirred at room temperature for 1 h. The resin is then

filtered off with suction and thoroughly washed with the cleavage solution. The combined filtrates are concentrated in vacuo and treated with ether. The precipitated solid is removed by filtration or centrifugation, washed with diethyl ether and dried under reduced pressure.

V. Purification and characterization of the peptides

Purification was carried out by gel chromatography (SEPHADEX G-10, G-15/10% HOAc, SEPHADEX LH20/MeOH) and/or medium pressure chromatography (stationary phase: HD-SIL C-8, 20-45 mikron, 100 Angstrom; mobile phase: gradient with A = 0.1% TFA/water, B = 0.1% TFA/MeOH), or preparative HPLC (stationary phase: Waters Delta-Pak C-18, 15 mikron, 100 Angstrom; mobile phase: gradient with A = 0.1 % TFA/water, B = 0.1 % TFA/MeOH).

The purity of the resulting products was determined by analytical HPLC (stationary phase: 100 2.1 mm VYDAC C-18, 300 Angstrom; mobile phase: acetonitrile-water gradient, buffered with 0.1% TFA, 40°C).
Characterization was by fast atom bombardment mass spectroscopy and NMR spectroscopy.

B. Specific procedures

EXAMPLE 1 (SEQ ID NO: 1)

$Me_2$Val-Val-MeVal-3,4-dehydroprolyl-Pro-amide

0.53 g of Fmoc-RINK-resin (substitution 0.46 mmol/g), corresponding to a batch size of 0.25 mmol, were reacted as in AIa with 0.4 mmol each of

Fmoc-Pro-OH
Fmoc-3,4-dehydroproline
Fmoc-MeVal-OH
Fmoc-Val-OH
Fmoc-Val-OH

The amino acid following the N-methylamino acid was coupled on with PyBrop as coupling reagent with a double coupling. After the iterative synthetic cycles were completed, the peptide-resin underwent N-terminal deprotection (steps 2-4 in AIa), and was further reacted with aqueous formaldehyde solution as in AII and then dried under reduced pressure. The resulting resin was subjected to TFA cleavage as in AIII. The crude product (132 mg) was purified by preparative medium pressure chromatography to yield 32 mg of the desired pure peptide (10-40% A in 10'; 40-90% A in 140'). The compound was further characterized by fast atom bombardment mass spectrometry ($[M+H]^+$ = 548).

EXAMPLE 2 (SEQ ID NO: 1)

$Me_2$Val-Val-MeVal-[(2S,3S)-3-methyl-pyrrolidine-2-carbonyl]-Pro-benzylamide

a) Z-(2S,3S)-3-Methyl-pyrollidine-2-carboxylic acid

1.5 g (2S,3S)-3-Methyl-pyrrolidine-2-carboxylic acid (11.6 mmol) were dissolved in 4 ml water and 2.33 ml 5 N NaOH. At 4°C, 2.12 ml benzylchloroformate (Z-C1; 12.8 mmol) and 6.5 ml 2 N NaOH were added within 1 h. After the reaction mixture was stirred overnight at room temperature, the pH was adjusted to 10. The aqueous layer was extracted with dichloromethane (4x), adjusted to pH 2 with 5 N HCl and extracted with dichloromethane (2x). The combined organic extracts were dried over sodium sulfate and evaporated in vacuo to yield 1.75 g of the desired product as an oil.

b) Z-(2S,3S)-3-Methyl-pyrrolidine-2-carbonyl-L-prolyl benzylamide

1.75 g (2S,3S)-3-Methyl-pyrrolidine-2-carboxylic acid (6.62 mmol) and 1.59 g proline benzylamide hydrochloride (6.62 mmol) were dissolved in 66 ml dichloromethane and cooled to 4°C. After addition of 0.89 ml N-methylmorpholine (7.94 mmol), 0.304 g HOBt (2.21 mmol) and 1.28 g EDCI (6.62 mmol), the reaction mixture was stirred at room temperature overnight, diluted with 150 ml dichloromethane, and washed with saturated sodium hydrogen carbonate solution (3x), water (1x), 5 % citric acid (3x), water (1x), and saturated NaCl (1x). The organic layer was dried over sodium sulfate and concentrated under reduced pressure to yield 2.63 g of a highly viscous oil.

c) (2S,3S)-3-Methyl-pyrrolidine-2-carbonyl-L-prolyl benzylamide

2.63 g Z-(2S,3S)-3-Methyl-pyrrolidine-2-carbonyl-L-prolyl benzylamide (5.8 mmol) were dissolved in 43 ml methanol. After addition of 105 mg 10 % Palladium/charcoal, the reaction mixture was hydrogenated overnight. Filtration and evaporation to dryness yielded 1.88 g of the deprotected dipeptide

d) Z-MeVal-[(2S,3S)-3-Methyl-pyrrolidine-2-carbonyl]-Probenzylamide

1.54 2-MeVal-OH (5.8 mmol) and 1.88 g (2S,3S)-3-Methylpyrrolidine-2-carbonyl-L-prolyl benzylamide (5.8 mmol) were dissolved in 58 ml dichloromethane and cooled to 4°C. After addition of 0.78 ml N-methylmorpholine (6.96 mmol), 0.266 g HOBt (1.93 mmol) and 1.12 g EDCI (5.8 mmol), the reaction mixture was stirred at room temperature overnight, diluted with 120 ml dichloromethane, and washed with sat. sodium hydrogen carbonate, once (3x), water (1x), 5 % citric acid (3x), water (1x), and sat. NaCl (1x). The organic layer was dried over sodium sulfate and concentrated under reduced pressure to yield 3.01 g of the product as a dry white foam.

e) MeVal-[(2S,3S)-3-Methyl-pyrrolidine-2-carbonyl]-Probenzylamide

3.01 g of Z-MeVal-[(2S,3S)-3-Methyl-pyrrolidine-2-carbonyl]-Pro-benzylamide (5.33 mmol) were dissolved in 39 ml methanol. After addition of 96 mg 10 % Palladium/charcoal, the reaction mixture was hydrogenated for 3 h. Filtration and evaporation to dryness yielded 2.18 g of the deprotected tripeptide as a clear oil.

f) Z-Val-MeVal-[(2S,3S)-3-Methyl-pyrrolidine-2-carbonyl]-Pro-benzylamide

1.28 g Z-Val-OH (5.11 mmol) were dissolved in 10 ml dichloromethane. After addition of 0.73 ml triethylamine (5.37 mmol) and cooling to -10°C, 0.66 ml pivaloylchloride (5.37 mmol) were added slowly. Stirring of the mixture at -10°C for 1.5 h was followed by addition of a solution of 2.18 g Meval-[(2S,3S)-3-Methyl-pyrrolidine-2-carbonyl]-Probenzylamide (5.11 mmol) and 0.73 ml triethylamine (5.37 mmol) in 10 ml dichloromethane. The reaction mixture was stirred at -10°C for 1.5 h and at room temperature overnight, diluted with dichloromethane, and washed with sat. sodium hydrogen carbonate (3x), water (1x), 5 % citric acid (3x), water (1x), and sat. NaCl (1x). The organic layer was dried over sodium sulfate and concentrated in vacuo to yield 2.75 g of the product as a dry foam.

g) Val-MeVal- [(2S,3S)-3-Methyl-pyrrolidine-2-carbonyl]-Pro-benzylamide

2.75 g of Z-Val-MeVal-[(2S,3S)-3-Methyl-pyrrolidine-2-carbonyl]-Pro-benzylamide (4.13 mmol) were dissolved in 30 ml methanol. After addition of 0.34 ml conc. HCl (4.13 mmol) and 78 mg 10 % Palladium/charcoal, the reaction mixture was hydrogenated for 3 h. Filtration and evaporation to dryness yielded 2.23 g of the deprotected tripeptide as a dry white foam. The crude product was further dissolved in 50 ml water and the pH adjusted to 2-3 with 1 N HCl. The aqueous layer was extracted with dichloromethane (3x), adjusted to pH 10 with 5 N NaOH, and further extracted with dichloromethane (3x). The combined organic extracts were dried over sodium sulfate and concentrated under reduced pressure to yield 1.71 g of the deprotected tetrapeptide benzylamide.

h) $Me_2$Val-Val-MeVal-[(2S,3S)-3-Methyl-pyrrolidine-2-carbonyl]-Pro-benzylamide

0.47 g $Me_2$Val-OH (3.24 mmol) and 1.71 g Val-MeVal-[(2S,3S)-3-Methyl-pyrrolidine-2-carbonyl]-Pro-benzylamide (3.24 mmol) were dissolved in 32 ml dry DMF. After cooling to 4°C, 1.07 ml DEPCN (6.48 mmol) and 1.88 ml triethylamine (12.96 mmol) were added. The reaction mixture was stirred at room temperature overnight and the DMF evaporated under reduced pressure. The residue was dissolved in 100 ml dichloromethane and washed with sat. sodium hydrogen carbonate (3x), water (1x), 5 % citric acid (3x), water (1x), and sat. NaCl (1x). The organic layer was dried over sodium sulfate and concentrated under reduced pressure to yield 0.88 g of the product as a colourless oil. The crude product was dissolved in boiling diisopropylether and precipitated upon cooling. The precipitate was collected and dried to yield 0.56 g of the pure product. The compound was further characterized by fast atom bombardment mass spectrometry ($[M+H]^+$ = 655.6).

The following compounds were prepared and can be prepared according to examples 1 and 2:

3. Xaa Val Xab Xac Xad
4. Xaa Val Xab Xac Xae
5. Xaa Val Xab Xac Xaf
6. Xaa Val Xab Xac Xag

7. Xaa Val Xab Xac Xah
8. Xaa Val Xab Xac Xai
9. Xaa Val Xab Xac Xak
10. Xaa Val Xab Xac Xal
11. Xaa Val Xab Xac Xam
12. Xaa Val Xab Xac Xan
13. Xaa Val Xab Xac Xao
14. Xaa Val Xab Xac Xap
15. Xaa Val Xab Xac Xaq
16. Xaa Val Xab Xac Xar
17. Xaa Val Xab Xac Xas
18. Xaa Val Xab Xac Xat
19. Xaa Val Xab Xac Xat
20. Xaa Val Xab Xac Xav
21. Xaa Val Xab Xac Xaw
22. Xaa Val Xab Xac Xax
23. Xaa Val Xab Xac Xay
24. Xaa Val Xab Xac Xaz
25. Xaa Val Xab Xac Xba
26. Xaa Val Xab Xac Xbb
27. Xaa Val Xab Xac Xbc
28. Xaa Val Xab Xac Xbd
29. Xaa Val Xab Xac Xbe
30. Xaa Val Xab Xac Xbf
31. Xbg Val Xab Xac Xar
32. Xbg Val Xab Xac Xas
33. Xbh Val Xab Xac Xar
34. Xbh Val Xab Xac Xas
35. Xaa Ile Xab Xac Xar
36. Xaa Ile Xab Xac Xas
37. Xaa Xbk Xab Xac Xar
38. Xaa Xbk Xab Xac Xas
39. Xaa Val Xbi Xac Xar
40. Xaa Val Xbi Xac Xas
41. Xaa Val Xab Xac Xbl
42. Xaa Val Xab Xac Xbm
43. Xaa Val Xab Xac Xbn
44. Xaa Val Xab Xbo Xae
45. Xaa Val Xab Xbo Xbp
46. Xaa Val Xab Xbo Xar
47. Xaa Val Xab Xbo Xas
48. Xaa Val Xab Xbo Xbm
49. Xaa Val Xab Xbq Xae
50. Xaa Val Xab Xbq Xar
51. Xaa Val Xab Xbq Xas
52. Xaa Val Xab Xbq Xbm
53. Xaa Val Xab Xbr Xbp
54. Xaa Val Xab Xbr Xae
55. Xaa Val Xab Xbr Xas
56. Xaa Val Xab Xbr Xar
57. Xaa Val Xab Xbs Xae
58. Xaa Val Xab Xbs Xas
59. Xaa Val Xab Xbs Xar
60. Xaa Val Xab Xac Xbu
61. Xaa Val Xab Xac Xbt
62. Xaa Val Xab Xbq Xbu
63. Xaa Val Xab Xbq Xbt
64. Xaa Val Xab Xac Pro Xbv

65. Xaa Val Xab Xac Pro Xbw
66. Xaa Val Xab Xac Pro Xbx
67. Xaa Val Xab Xac Pro Xby
68. Xaa Val Xab Xac Pro Xbz
64. Xaa Val Xab Xbq Pro Xbv
65. Xaa Val Xab Xbq Pro Xbw
66. Xaa Val Xab Xbq Pro Xbx
67. Xaa Val Xab Xbq Pro Xby
68. Xaa Val Xab Xbq Pro Xbz
69. Xaa Val Xab Xbo Xaz
70. Xaa Val Xab Xbq Xaz
71. Xaa Val Xab Xbq Xbn
72. Xaa Val Xab Xbo Xbn

Examples for the MS-characterization of the synthesized novel compounds are given in the following table.

| EXAMPLE | Fast atom bombardment MS analysis. |
|---|---|
| [No.] | [Mol.-Weight (measured)] |
| 45. | 627 |
| 53. | 655 |

Table I -

| Sequence Identification of Compounds Prepared According to Examples 1 and 2 | |
|---|---|
| Compound Number(s) | Sequence ID Number |
| 1-34, 39-63, 69-72 | 1 |
| 35, 36 | 2 |
| 37, 38 | 3 |
| 64-68 | 4 |

The symbols Xaa in the summary have the following meanings:

Xaa: N,N-Dimethylvaline

Xab: N-Methylvaline

Xac: 3,4-dehydroproline

Xad: 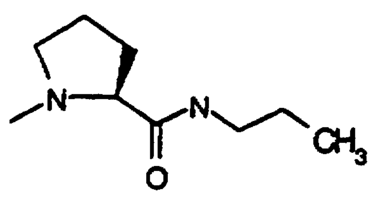

Xae: 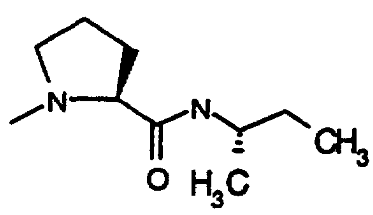

Xaf:

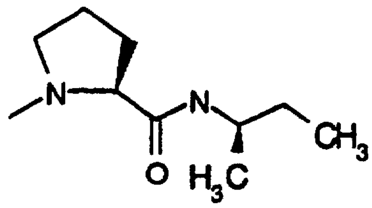

Xag:

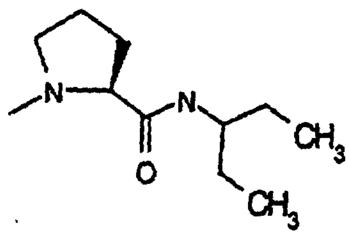

Xah:

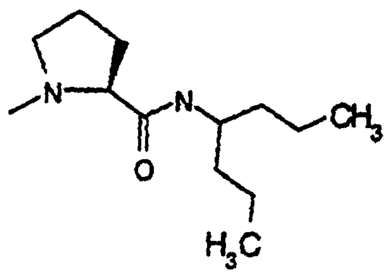

Xai:

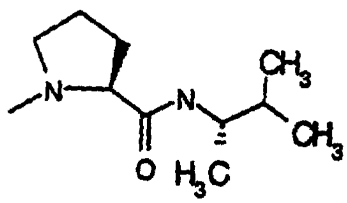

Xak:

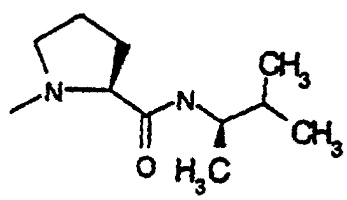

Xal:

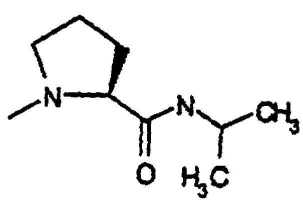

Xam: 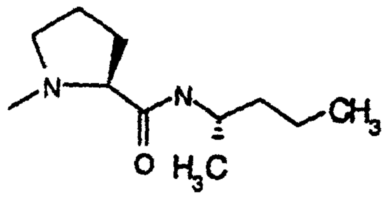

Xan: 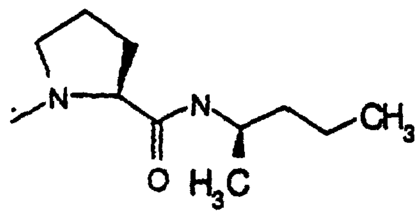

Xao: 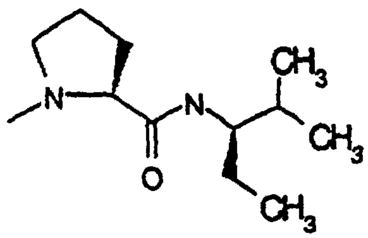

Xap: 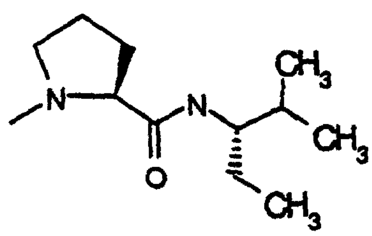

Xaq: 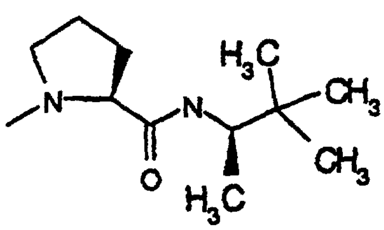

Xar: 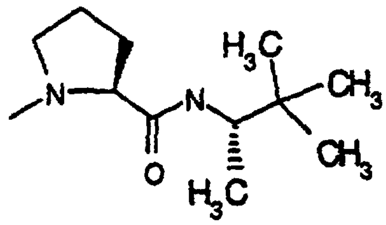

Xas: 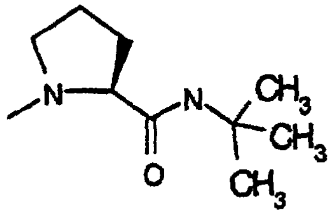

Xat: 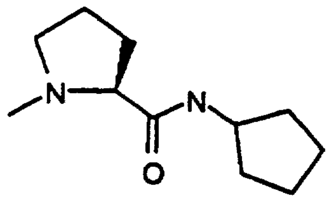

Xau: 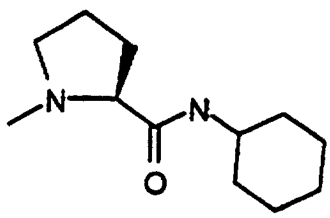

Xav: 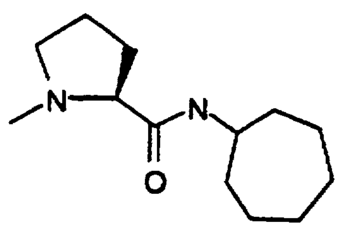

Xaw:

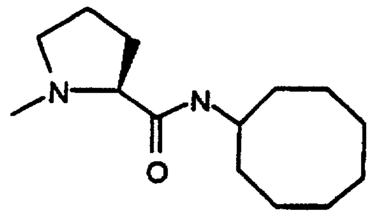

Xax:

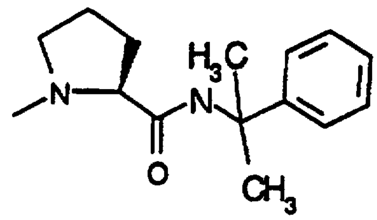

Xay:

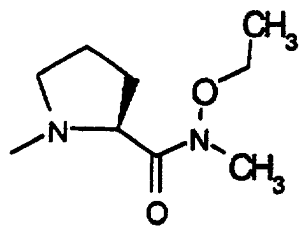

Xaz:

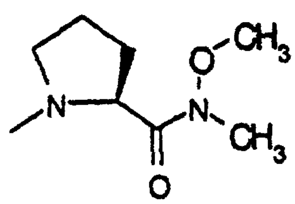

Xba:

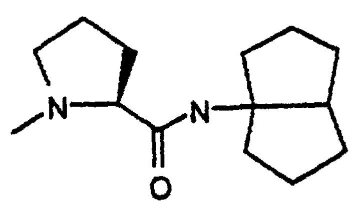

Xbb:

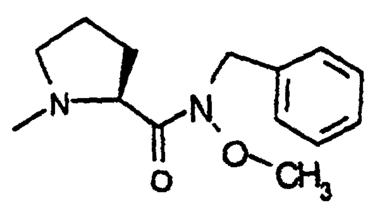

Xbc: 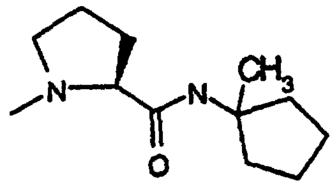

Xbd: 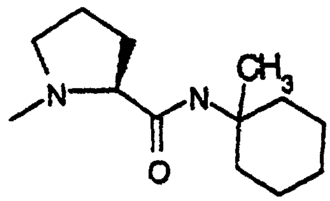

Xbe: 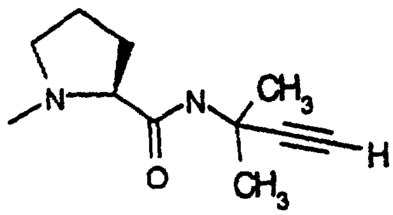

Xbf: 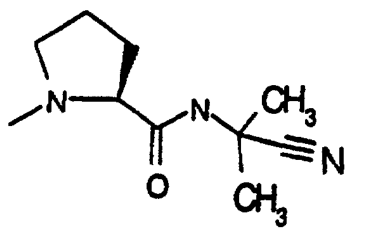

Xbg: N,N-dimethylisoleucine

Xbh: N,N-dimethyl-2-tert.butyl-glycine

Xbi: N-methylisoleucine

Xbk: 2-tert.butylglycine

Xbl: 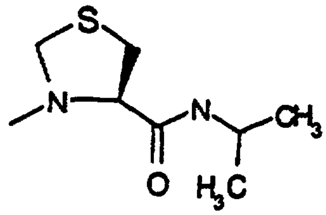

Xbm:

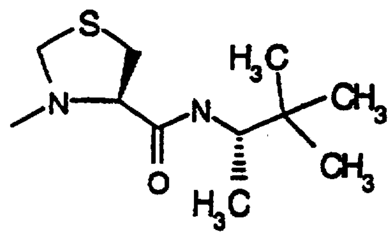

Xbn:

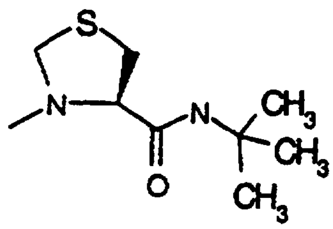

Xbo: L-azetidine-2-carbonyl

Xbp:

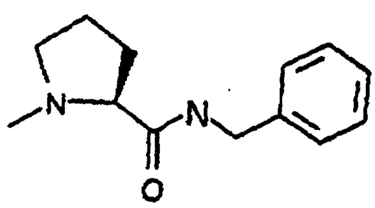

Xbq: (4-fluoro)-L-proline

Xbr: (5-methyl)-L-proline

Xbs: (3-methyl)-L-proline

Xbt:

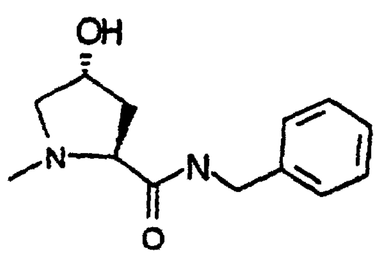

Xbu:

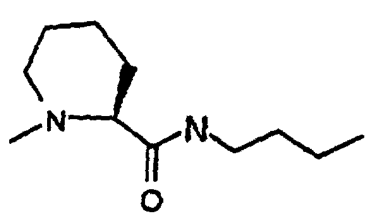

Xbv:

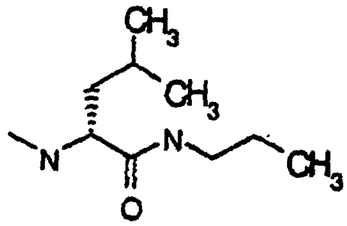

Xbw:

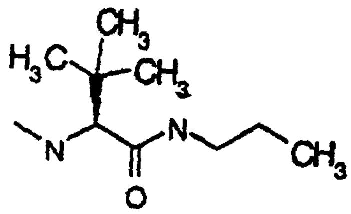

Xbx:

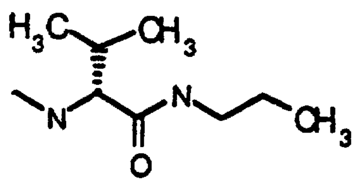

Xby:

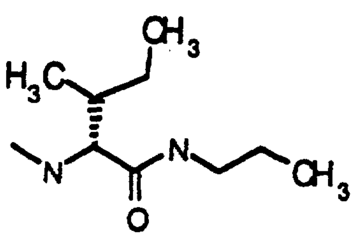

Xbz:

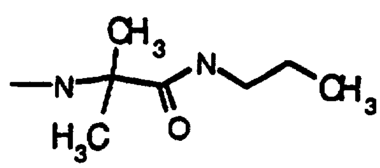

Compounds of this invention may be assayed for anti-cancer activity by conventional methods, including for example, the methods described below.

A. In vitro methodology

Cytotoxicity was measured using a standard methodology for adherent cell lines such as the microculture tetrazolium assay (MTT). Details of this assay have been published (Alley, MC et al, Cancer Research 48:589-601, 1988). Exponentially growing cultures of tumor cells such as the HT-29 colon carcinoma or LX-I lung tumor are used

to make microtiter plate cultures. Cells are seeded at 5000-20,000 cells per well in 96-well plates (in 150 µl of media), and grown overnight at 37°C. Test compounds are added, in 10-fold dilutions varying from $10^{-4}$ M to $10^{-10}$ M. Cells are then incubated for 48 hours. To determine the number of viable cells in each well, the MTT dye is added (50 µl of 3 mg/ml solution of 3-(4,5-dimethylthiazol-2-yl)-2,5-diphenyltetrazolium bromide in saline). This mixture is incubated at 37°C for 5 hours, and then 50 µl of 25 % SDS, pH2 is added to each well. After an overnight incubation, the absorbance of each well at 550 nm is read using an ELISA reader. The values for the mean +/- SD of data from replicated wells are calculated, using the formula % T/C (% viable cells treated/control).

$$\frac{\text{OD of treated cells}}{\text{OD of control cells}} \times 100 = \%\ \text{T/C}$$

The concentration of test compound which gives a T/C of 50% growth inhibition was designated as the $IC_{50}$ value.

| COMPOUND OF EXAMPLE | $IC_{50}$ [M] |
|---|---|
| 1. | $1 \times 10^{-9}$ |
| 2. | $1 \times 10^{-9}$ |
| 45. | $1 \times 10^{-7}$ |
| 53. | $1 \times 10^{-8}$ |

B. In vivo methodology

Compounds of this invention were further tested in preclinical assay for in vivo activity which is indicative of clinical utility. Such assays were conducted with nude mice into which tumor tissue, preferably of human origin, had been transplanted (xenografted), as is well known in this field. Test compounds were evaluated for their anti-tumor efficacy following administration to the xenograft-bearing mice.

More specifically, human breast tumors (MX-1) which had been grown in athymic nude mice were transplanted into new recipient mice, using tumor fragments which were about 50 mg in size. The day of transplantation was designated as day 0. Six to ten days later, mice were treated with the test compounds given as an intravenous injection, in groups of 5-10 mice at each dose. Compounds were given every other day, for 3 weeks, at doses from 1-100 mg/kg body weight. Tumor diameters and body weights were measured twice weekly. Tumor volumes were calculated using the diameters measured with Vernier calipers, and the formula

$$(\text{length} \times \text{width2})/2 = \text{mm}^3 \text{ of tumor volume}$$

Mean tumor volumes are calculated for each treatment group, and T/C values determined for each group relative to the untreated control tumors.

The new compounds possess good tumor inhibiting properties.

## Claims

**1.** Peptides of the formula I

$$R^1R^2N\text{-}CHX\text{-}CO\text{-}A\text{-}B\text{-}D\text{-}E\text{-}(F)_t\text{-}K \qquad I$$

where

| | |
|---|---|
| $R^1$ | is methyl, ethyl, or isopropyl; |
| $R^2$ | is hydrogen, methyl, or ethyl; |
| $R^1$-N-$R^2$ | together may be a pyrrollidine ring; |
| A | is a valyl, isoleucyl, leucyl, 2-tert-butylglycyl, 2- ethylglycyl, norleucyl or norvalyl residue; |
| B | is a N-methyl-valyl, -leucyl, -isoleucyl, -norvalyl, -norleucyl -2-tert-butylglycyl, -3-tert-butylalanyl, or -2-ethylglycyl residue; |
| D | is a 3,4-dehydroprolyl, 4-fluoroprolyl, 4,4-difluoroprolyl, azetidine-2-carbonyl, 3-methylprolyl, 4-methylprolyl, or 5-methylprolyl residue; |
| E | is a prolyl, homoprolyl, hydroxyprolyl or thiazolidine-4-carbonyl residue; |
| F | is a valyl, 2-tert-butylglycyl, isoleucyl, leucyl, 2-cyclohexylglycyl, norleucyl, norvalyl, neopentylglycyl, alanyl, β-alanyl, or aminoisobutyroyl residue; |
| X | is alkyl (preferably $C_{2-5}$), cyclopropyl, or cyclopentyl; |
| t | is 0 or 1; and |
| K | $C_{1-4}$-alkoxy, benzyloxy, $-NH_2$, or a substituted amino moiety which is $-NH-C_{1-12}$-alkyl, $-NH-C(CH_3)_2CN$, $-NH-C(CH_3)_2CCH$, $-NH-C(CH_3)_2C=CH_2$, $-NH-C(CH_3)_2CH_2CH_2OH$, $-NH-C(CH_3)_2CH_2OH$, $-NH-C_{3-8}$-cycloalkyl, -NH-[3,3,0]-bicyclooctyl, norephedryl, norpseudoephedryl, -NH-quinolyl, -NH-pyrazyl, $-NH-CH_2$-benzimidazolyl, -NH-adamantyl, $-NH-CH_2$-adamantyl, $-NH-CH(CH_3)$-phenyl, $-NH-C(CH_3)_2$-phenyl, $-N(C_{1-4}$-alkoxy)-$C_{1-4}$-alkyl, $-N(C_{1-4}$-alkoxy)-$CH_2$-phenyl, $-N(C_{1-4}$-alkoxy)-phenyl, $-N(CH_3)OBzl$, $-NH-(CH_2)_v$-phenyl (v=0,1,2, or 3), $-NH-(CH_2)_m$-naphthyl (m=0 or 1), $-NH-(CH_2)_w$-benzhydryl (w=0,1, or 2), -NH-biphenyl, -NH-pyridyl, $-NH-CH_2$-pyridyl, $-NH-CH_2-CH_2$-py- |

ridyl, -NH-benzothiazolyl, -NH-benzoisothiazolyl, -NH-benzopyrazolyl, -NH-benzoxazolyl, -NH-$(CH_2)_m$-fluorenyl (m=0 or 1), -NH-pyrimidyl, -NH-$(CH_2)_m$-indanyl (m=0 or 1), -NH-$(CH_2CH_2O)_y$-$CH_3$ (y=0,1,2,3,4, or 5), -NH-$(CH_2CH_2O)_y$-$CH_2CH_3$ (y=0,1,2,3,4, or 5), -$(CH_2CH_2O)_y$-$CH_3$ (y=0,1,2,3,4, or 5), -$(CH_2CH_2O)$y-$CH_2CH_3$ (y=0,1,2,3,4, or 5), -$NCH_3$-NH-$C_6H_5$, -$NCH_3$-NH-$CH_2$-$C_6H_5$; or K is

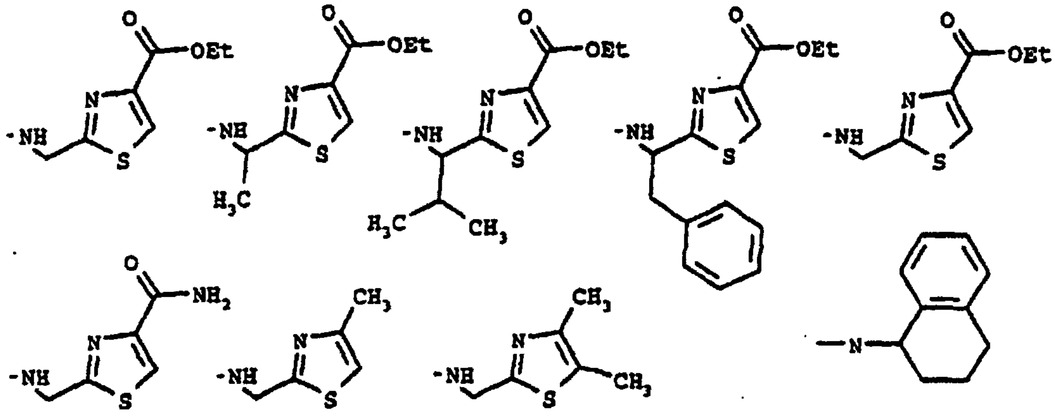

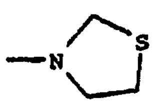
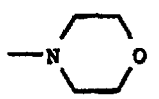
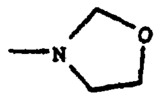
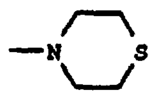
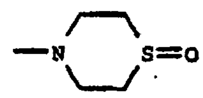

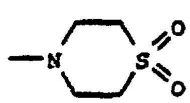
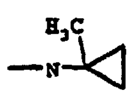
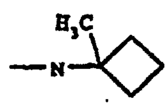
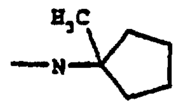

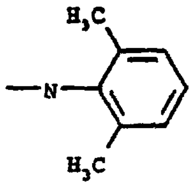
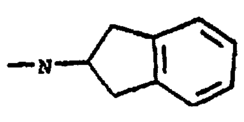
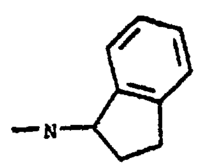
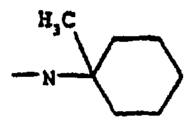

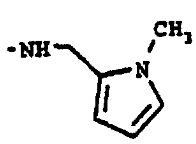
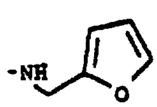
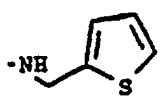
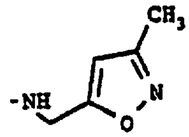
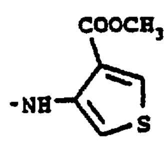

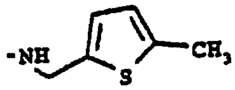 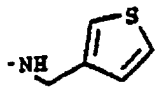 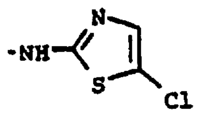 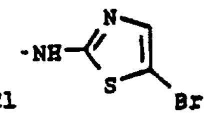 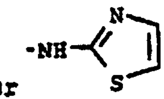

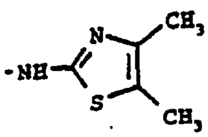 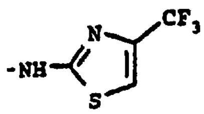 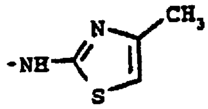 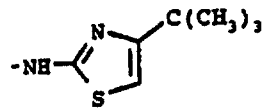

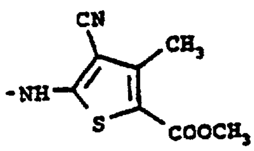 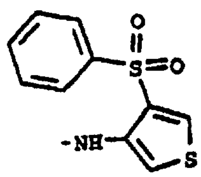 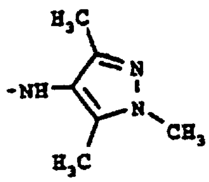 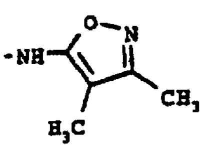

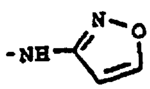 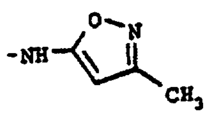 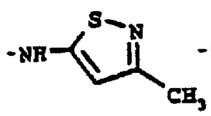 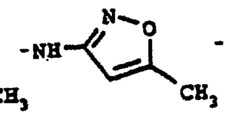 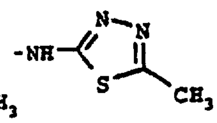

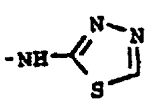 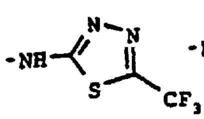 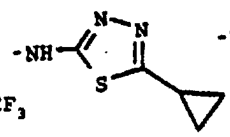 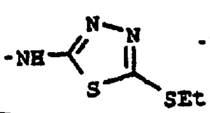 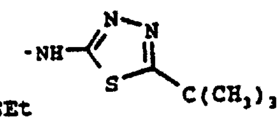

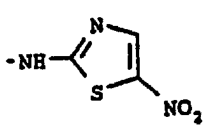 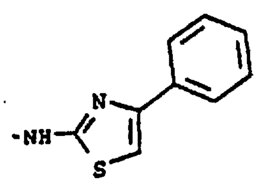 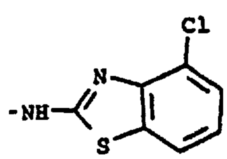 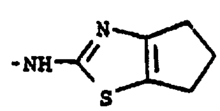

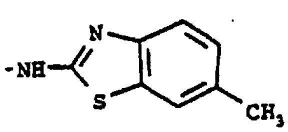 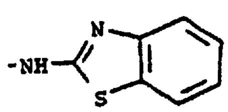 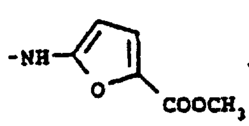 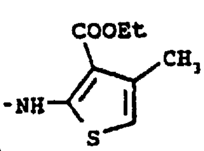

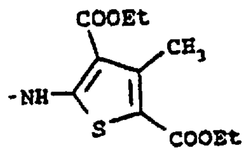 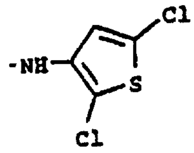 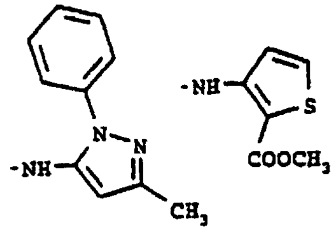 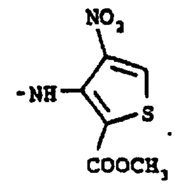

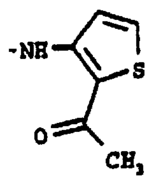 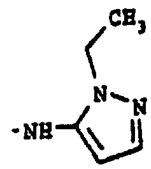 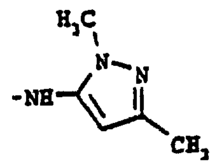 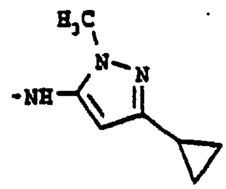

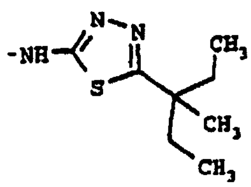 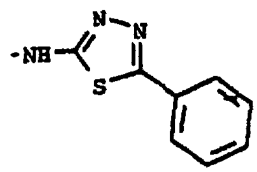 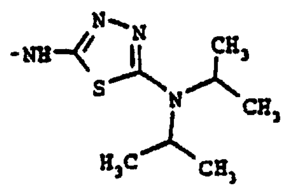

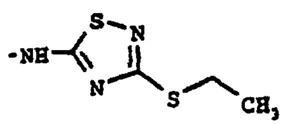 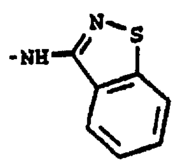 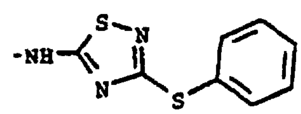

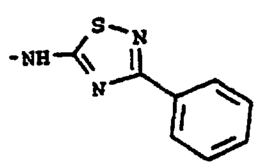 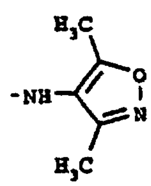 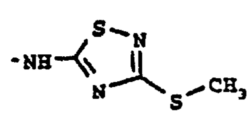

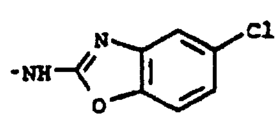 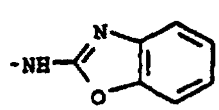 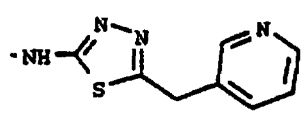

and the salts thereof with physiologically tolerated acids.

**2.** The compounds of claim 1, where

$R^1$ is methyl or ethyl;

R2 is hydrogen, methyl, or ethyl;

A is a valyl, isoleucyl, 2-tert-butylglycyl residue;

B is a N-methyl-valyl, -isoleucyl, or -2-tert-butylglycyl residue;

D is a 3,4-dehydroprolyl, 4-fluoroprolyl, azetidine-2-carbonyl, 3-methylprolyl, or 5-methyl-prolyl residue;

E is a prolyl, homoprolyl, hydroxyprolyl or thiazolidine-4-carbonyl residue;

F is a D-valyl, 2-tert-butylglycyl, D-isoleucyl, D-leucyl, or aminoisobutyroyl residue;

x is $-CH(CH_3)_2$, $-C(CH_3)_3$, or $-CH(CH_3)CH_2CH_3$;

t is 0 or 1.

**3.** The compounds of claim 1 or claim 2, where K is $-OC(CH_3)_3$, $-NHCH_3$, $-NHCH_2CH_3$, $-NH(CH_2)_2CH_3$, $-NH(CH_2)_3CH_3$, $-NH(CH_2)_4CH_3$, $-NH(CH_2)_5CH_3$, $-NH(CH_2)_6CH_3$, $-NH(CH_2)_7CH_3$, $-NHCH(CH_3)_2$, $-NHCH(CH_3)CH_2CH_3$, $-NHCH(CH_3)CH_2CH_2CH_3$, $-NHCH(CH_2CH_3)_2$, $-NH(CH_2CH_2CH_3)_2$, $-NHC(CH_3)_3$, $-NHCH(CH_2CH_3)CH_2CH_2CH_3$, $-NHCH(CH_3)CH(CH_3)_2$, $-NHCH(CH_2CH_3)CH(CH_3)_2$, $-NHCH(CH_3)C(CH_3)_3$, -NH-cyclopropyl, -NH-cyclobutyl, -NH-cyclopentyl, -NH-cyclohexyl, -NH-cycloheptyl, -NH-cyclooctyl, -NH-bicyclo[3,3,0]octyl, $-N(CH_3)OCH_3$, $-N(CH_3)OCH_2CH_3$, $-M(CH_3)OCH_2CH_2CH_3$, $-N(CH_3)OCH(CH_3)_2$, $-N(CH_2CH_3)OCH_3$, $-N(CH_2CH_3)OCH_2H_3$, $-N(CH(CH_3)_2)OCH_3$, $-N(CH_3)OCH_2C_6H_5$, $-N(OCH_3)CH_2-C_6H_5$, $-N(CH_3)OC_6H_5$, $-NH-CH_2-C_6H_5$, $-NH(CH_2)_2C_6H_5$, $-NH(CH_2)_3C_6H_5$, $-NHCH(CH_3)C_6H_5$, $-NHC(CH_3)_2C_6H_5$, $-NHC(CH_3)_2CH_2CH_3$, $-NHC(CH_3)(CH_2CH_3)_2$, $-NHCH(CH_3)CH(OH)C_6H_5$, $-NHCH_2$-cyclohexyl, $-NH-CH_2CF_3$, $-NHCH(CH_2F)_2$, $-NHC(CH_3)_2CH_2CH_2OH$, $-NH(CH_2CH_2O)_2CH_2CH_3$, $-NHC(CH_3)_2CH(CH_3)_2$, $-NHC(CH_3)_2CN$, $-NHC(CH_3)_2CCH$, norephedryl, norpseudoephedryl, -NH-quinolyl, -NH-pyrazyl, -NH-adamantyl(2), -NH-adamantyl(1), $-NH-CH_2$-adamantyl, $-NH-CH_2$-naphthyl, -NH-benzhydryl, -NH-biphenyl, -NH-pyridyl, $-NH-CH_2$-pyridyl, $-NH-CH_2-CH_2$-pyridyl, -NH-benzothiazolyl, -NH-benzoisothiazolyl, -NH-benzopyrazolyl, -NH-benzoxazolyl, -NH-fluorenyl, -NH-pyrimidyl, $-NH-CH_2$-(4-methyl)-thiazolyl(2), $-NH-CH_2$-furanyl(2), $-NH-CH_2$-thienyl(2), $-NH-CH_2$-(5-methyl)thienyl(2), -NH-thiazolyl(2), -NH-isoxazolyl(3), -NH-(3-methyl)isoxazolyl(5), -NH-(3-methyl)isothiazolyl(5), -NH-(2-trifluormethyl)-thiadiazolyl(5), -NH-(2-cyclopropyl)thiadiazolyl(5), $-NHC(CH_3)_2C=CH_2$, or K may be

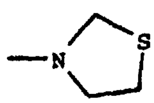 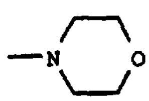 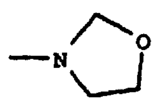 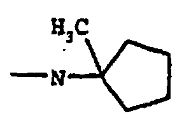 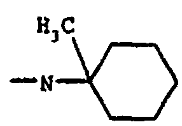

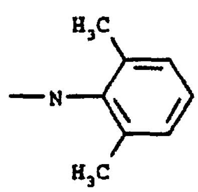 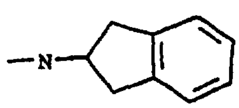 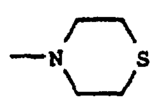 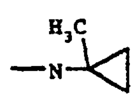 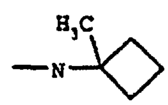

**4.** The compounds of any of claims 1 to 3 for use in medicine in particular for treating oncological diseases.

**5.** A pharmaceutical composition comprising a pharmaceutically acceptable carrier and a therapeutically effective amount of a compound of any of claims 1 to 3.

**6.** Use of a compound of any of claims 1 to 3 for preparing a pharmaceutical composition for treating a tumor in a mammal.

**7.** A method for preparing compounds of any of claims 1 to 3, **characterized in that** they are prepared according to known methods of peptide chemistry.

## Patentansprüche

**1.** Peptide der Formel I

$$R^1R^2N\text{-}CHX\text{-}CO\text{-}A\text{-}B\text{-}D\text{-}E\text{-}(F)_t\text{-}K \quad I$$

worin

$R^1$ für Methyl, Ethyl oder Isopropyl steht;

R2 für Wasserstoff, Methyl oder Ethyl steht;

$R^1$-N-$R^2$ zusammen einen Pyrrolidin-Ring bilden können;

A für einen Valyl-, Isoleucyl-, Leucyl-, 2-tert-Butylglycyl-, 2-Ethylglycyl-, Norleucyl- oder Norvalyl-Rest steht;

B für einen N-Methyl-valyl-, -leucyl-, -isoleucyl-, -norvalyl-, -norleucyl-, -2-tert-butylglycyl-, -3-tert-butylalanyl- oder -2-ethylglycyl-Rest steht;

D für einen 3,4-Dehydroprolyl-, 4-Fluorprolyl-, 4,4-Difluorprolyl-, Azetidin-2-carbonyl-, 3-Methylprolyl-, 4-Methylprolyl- oder 5-Methylprolyl-Rest steht;

E für einen Prolyl-, Homoprolyl-, Hydroxyprolyl- oder Thiazolidin-4-carbonyl-Rest steht;

F für einen Valyl-, 2-tert-Butylglycyl-, Isoleucyl-, Leucyl-, 2-Cyclohexylglycyl-, Norleucyl-, Norvalyl-, Neopentylglycyl-, Alanyl-, β-Alanyl- oder Aminoisobutyroyl-Rest steht;

x für Alkyl (vorzugsweise $C_{2-5}$), Cyclopropyl oder Cyclopentyl steht;

t für 0 oder 1 steht; und

K für $C_{1-4}$-Alkoxy, Benzyloxy, -$NH_2$ oder eine substituierte Aminoeinheit steht, nämlich -NH-$C_{1-12}$-Alkyl, -NH-$C(CH_3)_2CN$, -NH-$C(CH_3)_2CCH$, -NH-$C(CH_3)_2C{=}CH_2$, -NH-$C(CH_3)_2CH_2CH_2OH$, -NH-$C(CH_3)_2CH_2OH$, -NH-$C_{3-8}$-Cycloalkyl, -NH-[3,3,0]-Bicyclooctyl, Norephedryl, Norpseudoephedryl, -NH-Chinolyl, -NH-Pyrazyl, -NH-$CH_2$-Benzimidazolyl, -NH-Adamantyl, -NH-$CH_2$-Adamantyl, NH-$CH(CH_3)$-Phenyl, -NH-$C(CH_3)_2$-Phenyl, -N($C_{1-4}$-Alkoxy)-$C_{1-4}$-Alkyl, -N($C_{1-4}$-Alkoxy) -$CH_2$-phenyl, -N($C_{1-4}$-Alkoxy)-phenyl, -N($CH_3$)OBzl, -NH-$(CH_2)_v$-Phenyl (v = 0,1,2, oder 3), -NH-$(CH_2)_m$-Naphthyl (m = 0 oder 1), -NH-$(CH_2)_w$-Benzhydryl (w = 0,1, oder 2), -NH-Biphenyl, -NH-Pyridyl, -NH-$CH_2$-Pyridyl, -NH-$CH_2$-$CH_2$-Pyridyl, -NH-Benzothiazolyl, -NH-Benzoisothiazolyl, -NH-Benzopyrazolyl, -NH-Benzoxazolyl, -NH-$(CH_2)_m$-Fluorenyl (m = 0 oder 1), -NH-Pyrimidyl, -NH-$(CH_2)_m$-Indanyl (m = 0 oder 1), -NH-$(CH_2CH_2O)_y$-$CH_3$ (y = 0,1,2,3,4 oder 5), -NH-$(CH_2CH_2O)_y$-$CH_2CH_3$ (y = 0, 1, 2, 3, 4, oder 5), ), -$(CH_2CH_2O)_y$-$CH_3$ (y = 0,1,2,3,4 oder 5), -$(CH_2CH_2O)_y$-$CH_2CH_3$ (y = 0, 1, 2, 3, 4, oder 5), -$NCH_3$-NH-$C_6H_5$, -$NCH_3$-NH-$CH_2$-$C_6H_5$;

oder K für

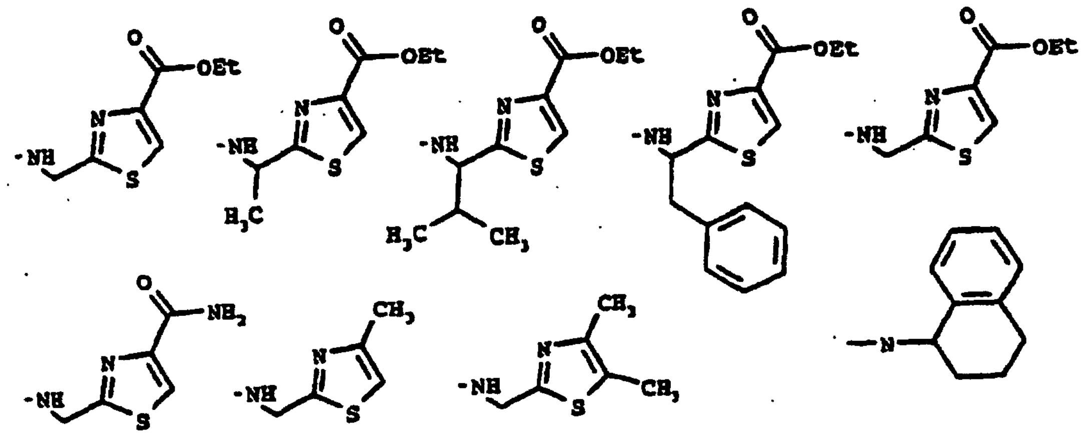

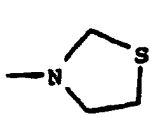 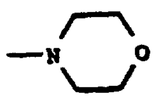 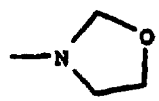 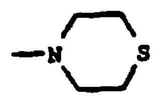 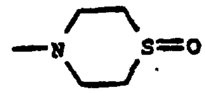

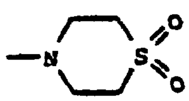 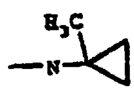 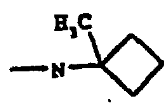 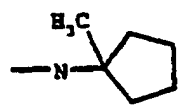

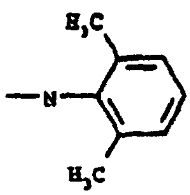 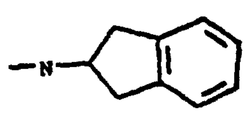 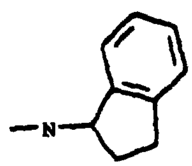 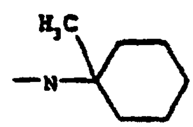

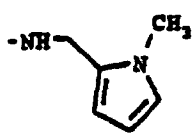 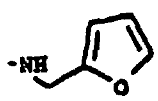 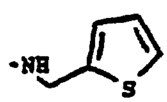 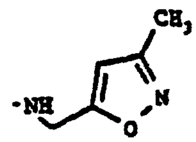 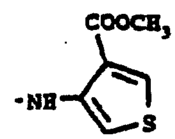

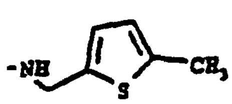 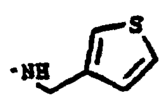 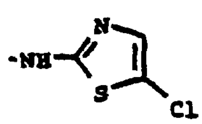 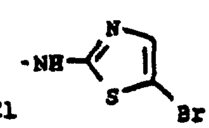 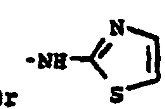

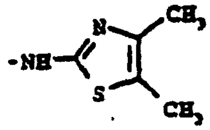 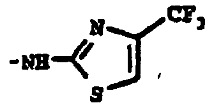 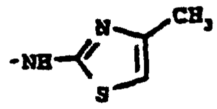 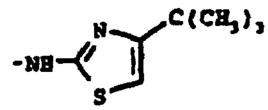

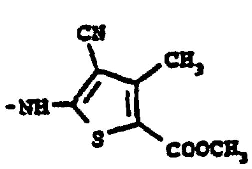 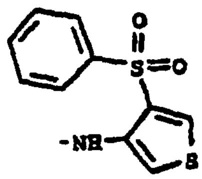 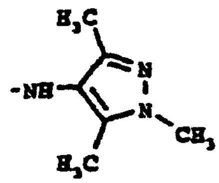 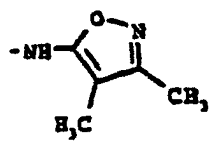

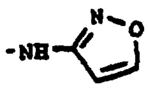 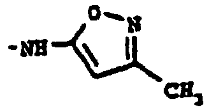 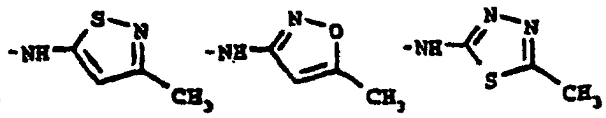

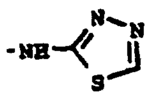 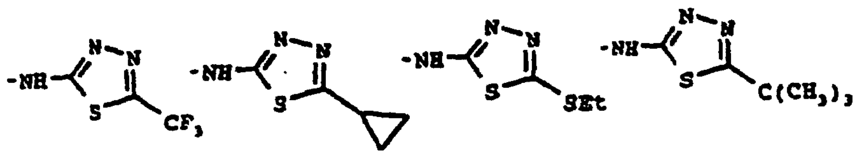

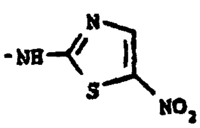 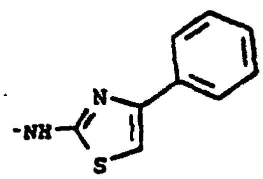 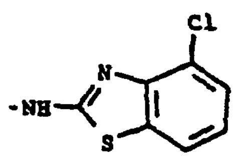 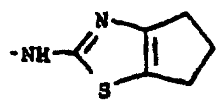

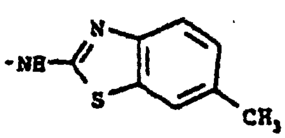 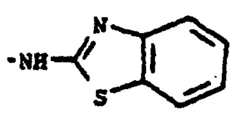 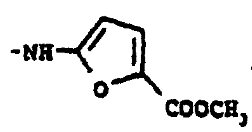 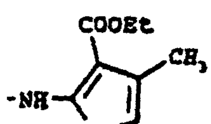

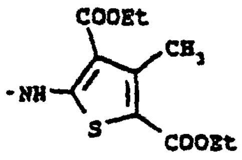
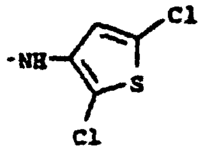
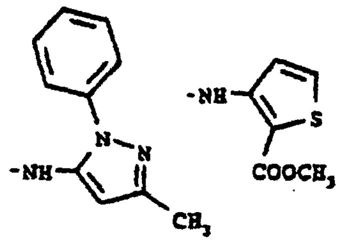
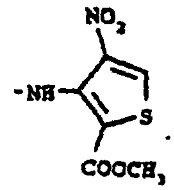

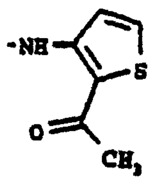
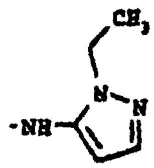
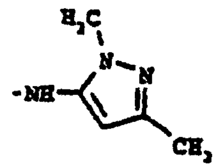
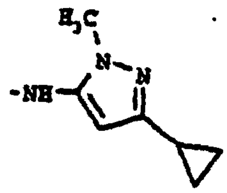

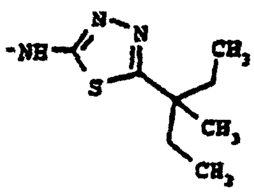
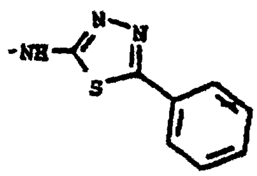
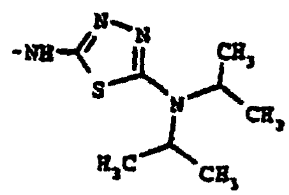

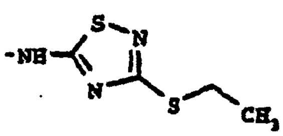
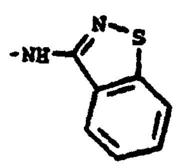
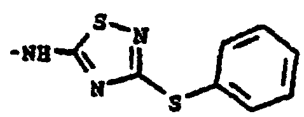

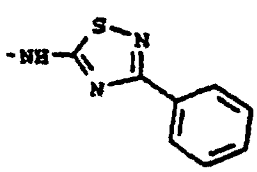
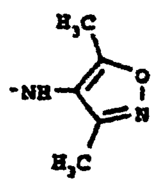
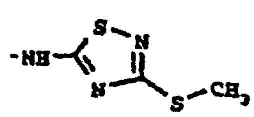

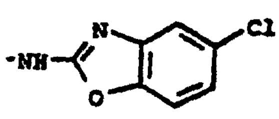
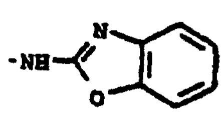
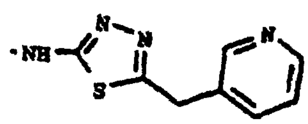

steht,
und die Salze davon mit physiologisch akzeptablen Säuren.

**2.** Verbindungen nach Anspruch 1, worin

$R^1$ Methyl oder Ethyl ist;

R2 Wasserstoff, Methyl oder Ethyl ist;

A ein Valyl-, Isoleucyl, 2-tert-Butylglycyl-Rest ist;

B ein N-Methyl-valyl-, -isoleucyl-, oder2-tert-butylglycyl-Rest ist;

D ein 3,4-Dehydroprolyl-, 4-Fluorprolyl-, Azetidin-2-carbonyl-, 3-Methylprolyl- oder 5-Methylprolyl-Rest ist;

E ein Prolyl-, Homoprolyl-, Hydroxyprolyl- oder Thiazolidin-4-carbonyl-Rest ist;

F ein D-Valyl-, 2-tert-Butylglycyl-, D-Isoleucyl-, D-Leucyl- oder Aminoisobutyroyl-Rest ist;

x $-CH(CH_3)_2$, $-C(CH_3)_3$ oder $-CH(CH_3)CH_2CH_3$ ist;

t 0 oder 1 ist.

**3.** Verbindung nach Anspruch 1 oder 2, worin K für
$-OC(CH_3)_3$, $-NHCH_3$, $-NHCH_2CH_3$, $-NH(CH_2)_2CH_3$, $-NH(CH_2)_3CH_3$, $-NH(CH_2)_4CH_3$, $-NH(CH_2)_5CH_3$, $-NH(CH_2)_6CH_3$, $-NH(CH_2)_7CH_3$, $-NHCH(CH_3)_2$, $-NHCH(CH_3)CH_2CH_3$, $-NHCH(CH_3)CH_2CH_2CH_3$, $-NHCH(CH_2CH_3)_2$, $-NH(CH_2CH_2CH_3)_2$, $-NHC(CH_3)_3$, $-NHCH(CH_2CH_3)CH_2CH_2CH_3$, $-NHCH(CH_3)CH(CH_3)_2$, $-NHCH(CH_2CH_3)CH(CH_3)_2$, $-NHCH(CH_3)C(CH_3)_3$, -NH-Cyclopropyl, -NH-Cyclobutyl, -NH-Cyclopentyl, -NH-Cyclohexyl, -NHCycloheptyl, -NH-Cyclooctyl, -NH-Bicyclo[3,3,0]octyl, $-N(CH_3)OCH_3$, $-N(CH_3)OCH_2CH_3$, $-N(CH_3)OCH_2CH_2CH_3$, $-N(CH_3)OCH(CH_3)_2$, $-N(CH_2CH_3)OCH_3$, $-N(CH_2CH_3)OCH_2CH_3$, $-N(CH(CH_3)_2)OCH_3$, $-N(CH_3)OCH_2C_6H_5$, $-N(OCH_3)CH_2C_6H_5$, $-N(CH_3)OC_6H_5$, $-NH-CH_2-C_6H_5$, $-NH(CH_2)_2C_6H_5$, $-NH(CH_2)_3C_6H_5$, $-NHCH(CH_3)C_6H_5$, $-NHC(CH_3)_2C_6H_5$, $-NHC(CH_3)_2CH_2CH_3$, $-NHC(CH_3)(CH_2CH_3)_2$, $-NHCH(CH_3)CH(OH)C_6H_5$, $-NHCH_2$-Cyclohexyl, $-NH-CH_2CF_3$, $-NHCH(CH_2F)_2$, $-NHC(CH_3)_2CH_2CH_2OH$, $-NH(CH_2CH_2O)_2CH_2CH_3$, $-NHC(CH_3)_2CH(CH_3)_2$, $-NHC(CH_3)_2CN$, $NHC(CH_3)_2CCH$, Norephedryl, Norpseudoephedryl, -NH-Chinolyl, -NH-Pyrazyl, -NH-Adamantyl(2), -NH-Adamantyl(1), $-NH-CH_2$-Adamantyl, $-NH-CH_2$-Naphthyl, -NH-Benzhydryl, -NH-Biphenyl, -NH-Pyridyl, $-NH-CH_2$-Pyridyl, $-NH-CH_2CH_2$-Pyridyl, -NH-Benzothiazolyl, -NH-Benzoisothiazolyl, -NH-Benzopyrazolyl, -NH-Benzoxazolyl, -NH-Fluorenyl, -NH-Pyrimidyl, $-NH-CH_2$-(4-Methyl)-thiazolyl(2), $-NH-CH_2$-Furanyl(2), $-NH-CH_2$-Thienyl(2), $-NH-CH_2$-(5-Methyl)thienyl(2), -NH-Thiazolyl(2), -NH-Isoxazotyl(3), -NH-(3-Methyl)isoxazolyl(5), -NH-(3-Methyl)isothiazolyl(5), -NH-(2-Trifluormethyl)-thiadiazolyl(5), -NH-(2-Cyclopropyl)thiadiazolyl(5), $-NHC(CH_3)_2C{=}CH_2$,
steht oder K

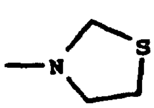
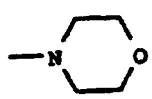
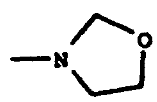
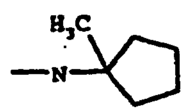
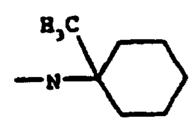

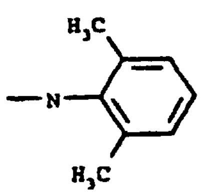
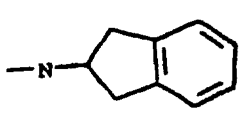
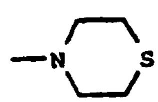
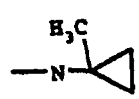
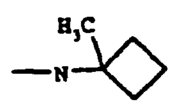

sein kann.

**4.** Verbindung nach einem der Ansprüche 1 bis 3 zur medizinischen Verwendung insbesondere für die Behandlung onkologischer Erkrankungen.

**5.** Pharmazeutische Zusammensetzung, umfassend einen pharmazeutisch verträglichen Träger und eine therapeutisch wirksame Menge einer Verbindung nach einem der Ansprüche 1 bis 3.

6. Verwendung einer Verbindung nach einem der Ansprüche 1 bis 3 zur Herstellung einer pharmazeutischen Zusammensetzung zur Behandlung eines Tumors in einem Säuger.

7. Verfahren zur Herstellung von Verbindungen nach einem der Ansprüche 1 bis 3, **dadurch gekennzeichnet, dass** man sie in Anlehnung an bekannte peptidchemische Verfahren herstellt.

## Revendications

1. Peptides de formule I

$$R^1R^2N\text{-CHX-CO-A-B-D-E-(F)}_t\text{-K} \qquad \text{I}$$

dans laquelle

| | |
|---|---|
| $R^1$ | est un groupe méthyle, éthyle ou isopropyle; |
| $R^2$ | est un atome d'hydrogène ou un groupe méthyle ou éthyle; |
| $R^1$-N-$R^2$ | peut former ensemble un cycle pyrrolidine; |
| A | est un résidu valyle, isoleucyle, leucyle, 2-tert-butylglycyle, 2-éthylglycyle, norleucyle ou norvalyle; |
| B | est un résidu N-méthylvalyle, -leucyle, -isoleucyle, -norvalyle, -norleucyle, -2-tert-butylglycyle, -3-tert-butylalanyle ou -2-éthylglycyle; |
| D | est un résidu 3,4-déhydroprolyle, 4-fluoroprolyle, 4,4-difluoroprolyle, azétidine-2-carbonyle, 3-méthylprolyle, 4-méthylprolyle ou 5-méthylprolyle; |
| E | est un résidu prolyle, homoprolyle, hydroxyprolyle ou thiazolidine-4-carbonyle; |
| F | est un résidu valyle, 2-tert-butylglycyle, isoleucyle, leucyle, 2-cyclohexylglycyle, norleucyle, norvalyle, néopentylglycyle, alanyle, β-alanyle ou aminoisobutyryle; |
| X | est un groupe alkyle (de préférence en $C_2$-$C_5$), cyclopropyle ou cyclopentyle; |
| t | est 0 ou 1; et |
| K | est un groupe alcoxy en $C_1$-$C_4$, benzyloxy, -$NH_2$ ou un groupe amino substitué qui est -NH-alkyle en $C_{1-12}$, -NH-$C(CH_3)_2CN$, -NH-$C(CR_3)_2CCH$, -NH-$C(CH_3)_2C{=}CH_2$, -NH-$C(CH_3)_2CH_2CH_2OH$, -NH-$C(CH_3)_2CH_2OH$, -NH-cycloalkyle en $C_3$-$C_8$, -NH-bicyclo[3.3.0]octyle, noréphédryle, norpseudoéphédryle, -NH-quinoléyle, -NH-pyrazyle, -NH-$CH_2$-benzimidazolyle, -NH-adamantyle, -NH-$CH_2$-adamantyle, -NH-$CH(CH_3)$-phényle, -NH-$C(CH_3)_2$-phényle, -N(alcoxy en $C_1$-$C_4$)-alkyle en $C_1$-$C_4$, -N(alcoxy en $C_1$-$C_4$)-$CH_2$-phényle, -N(alcoxy en $C_1$-$C_4$)-phényle, -N($CH_3$)OBzl, -NH-$(CH_2)_v$-phényle (v = 0, 1, 2 ou 3), -NH-$(CH_2)_m$-naphtyle (m = 0 ou 1), -NH-$(CH_2)_w$-benzhydryle (w = 0, 1 ou 2), -NH-biphényle, -NH-pyridyle, -NH-$CH_2$-pyridyle, - NH-$CH_2$-$CH_2$-pyridyle, -NH-benzothiazolyle, -NH-benzoisothiazolyle, -NH-benzopyrazolyle, -NH-benzoxazolyle, -NH-$(CH_2)_m$-fluorényle (m = 0 ou 1), -NH-pyrimidyle, -NH-$(CH_2)_m$-indanyle (m = 0 ou 1), -NH-$(CH_2CH_2O)_y$-$CH_3$ (y = 0, 1, 2, 3, 4 ou 5), -NH-$(CH_2CH_2O)_y$-$CH_2CH_3$ (y = 0, 1, 2, 3, 4 ou 5), -$(CH_2CH_2O)_y$-$CH_3$ (y = 0, 1, 2, 3, 4 ou 5), -$(CH_2CH_2O)_y$-$CH_2CH_3$ (y = 0, 1, 2, 3, 4 ou 5), -$NCH_3$-NH-$C_6H_5$, -$NCH_3$-NH-$CH_2$-$C_6H_5$; ou K est |

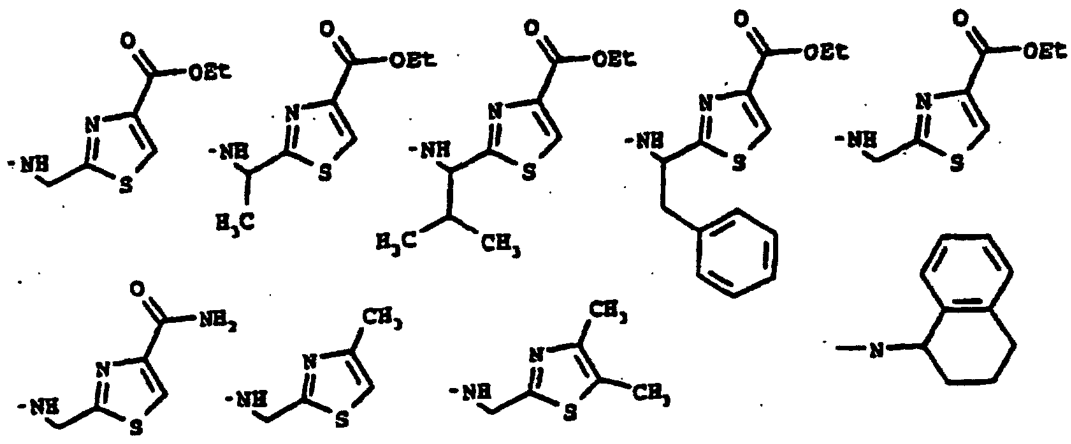

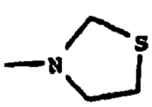
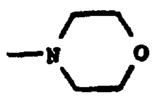
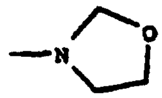
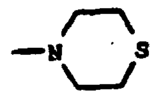
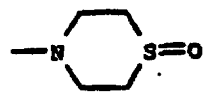

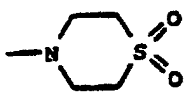
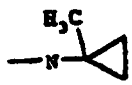
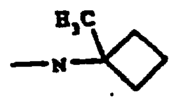
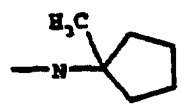

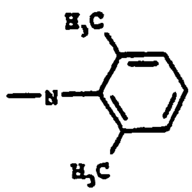
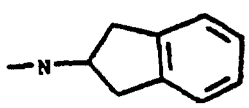
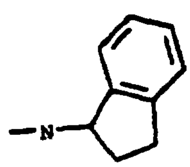
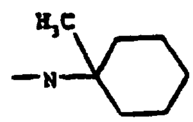

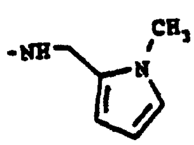
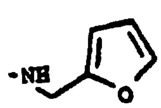
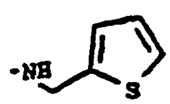
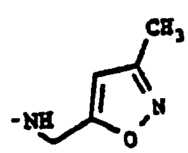
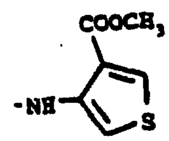

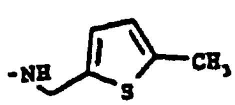
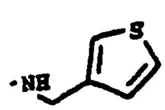
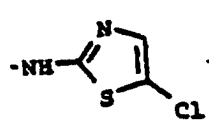
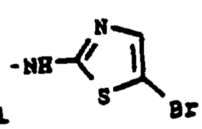
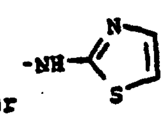

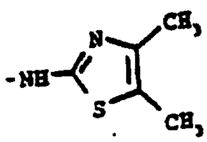 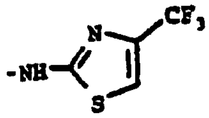 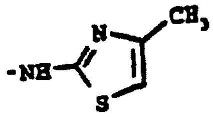 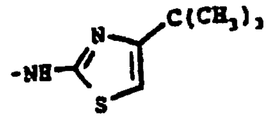

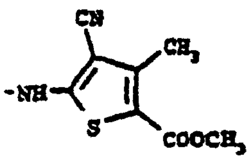 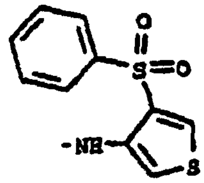 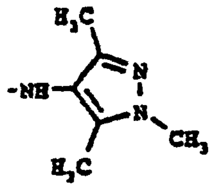 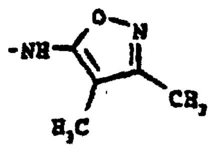

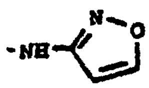 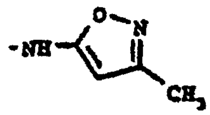 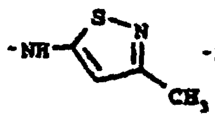 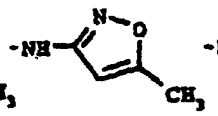 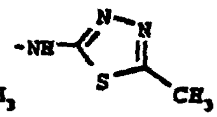

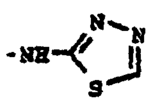 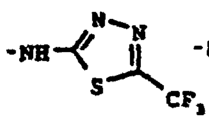 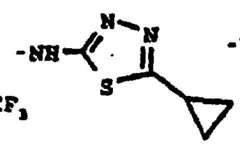 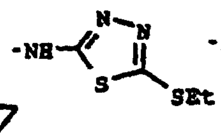 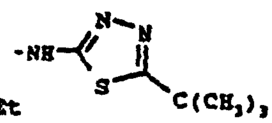

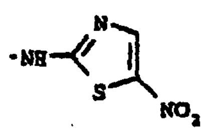 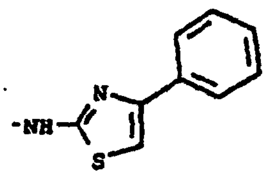 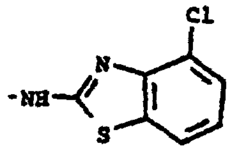 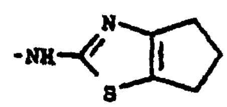

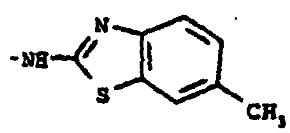 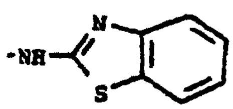 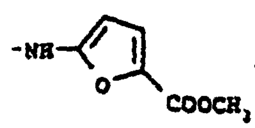 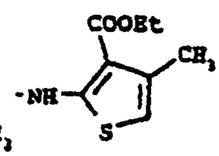

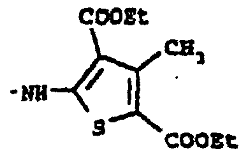
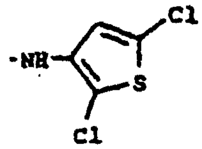
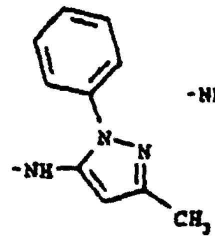
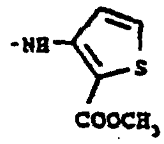
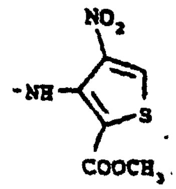

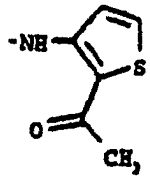
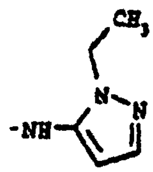
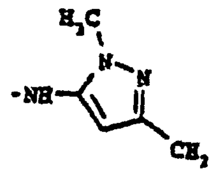
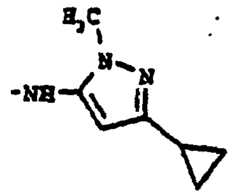

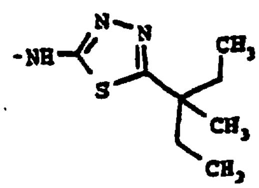
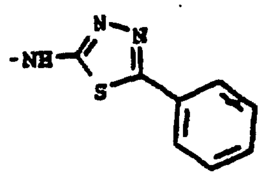
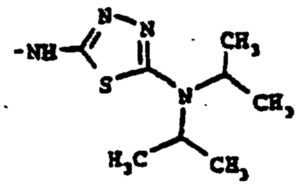

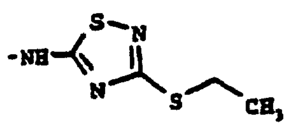
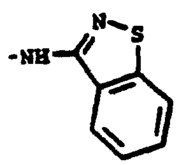
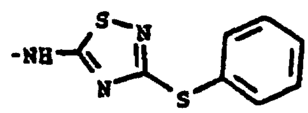

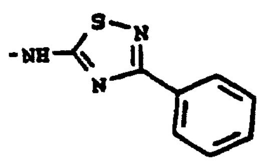
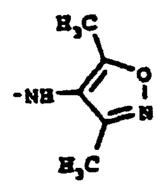
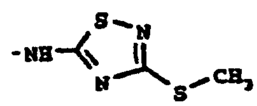

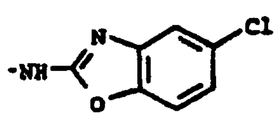
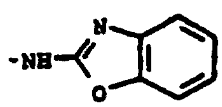
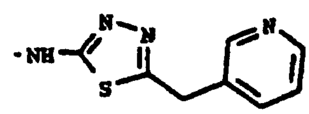

et leurs sels avec des acides tolérés physiologiquement.

**2.** Composés selon la revendication 1, dans lesquels

- $R^1$ est un groupe méthyle ou éthyle;
- $R^2$ est un atome d'hydrogène ou un groupe méthyle ou éthyle;
- A est un résidu valyle, isoleucyle ou 2-tert-butylglycyle;
- B est un résidu N-méthylvalyle, -isoleucyle ou -2-tert-butylglycyle;
- D est un résidu 3,4-déhydroprolyle, 4-fluoroprolyle, azétidine-2-carbonyle, 3-méthylprolyle ou 5-méthylprolyle;

E est un résidu prolyle, homoprolyle, hydroxyprolyle ou thiazolidine-4-carbonyle;
F est un résidu D-valyle, 2-tert-butylglycyle, D-isoleucyle, D-leucyle, ou aminoisobutyryle;
X est un groupe $-CH(CH_3)_2$, $-C(CH_3)_3$ ou $-CH(CH_3)CH_2CH_3$;
t est 0 ou 1.

**3.** Composés selon la revendication 1 ou la revendication 2, dans lesquels K est $-OC(CH_3)_3$, $-NHCH_3$, $-NHCH_2CH_3$, $-NH(CH_2)_2CH_3$, $-NH(CH_2)_3CH_3$, $-NH(CH_2)_4CH_3$, $-NH(CH_2)_5CH_3$, $-NH(CH_2)_6CH_3$, $-NH(CH_2)_7CH_3$, $-NHCH(CH_3)_2$, $-NHCH(CH_3)CH_2CH_3$, $-NHCH(CH_3)CH_2CH_2CH_3$, $-NHCH(CH_2CH_3)_2$, $-NH(CH_2CH_2CH_3)_2$, $-NHC(CH_3)_3$, $-NH-CH(CH_2CH_3)CH_2CH_2CH_3$, $NHCH(CH_3)CH(CH_3)_2$, $-NHCH(CH_2CH_3)CH(CH_3)_2$, $-NHCH(CH_3)C(CH_3)_3$, -NH-cyclopropyle, -NH-cyclobutyle, -NH-cyclopentyle, -NH-cyclohexyle, -NH-cycloheptyle, -NH-cyclooctyle, -NH-bicyclo[3.3.0]octyle, $-N(CH_3)OCH_3$, $-N(CH_3)OCH_2CH_3$, $-N(CH_3)OCH_2CH_2CH_3$, $-N(CH_3)OCH(CH_3)_2$, $-N(CH_2CH_3)OCH_3$, $-N(CH_2CH_3)OCH_2CH_3$, $-N(CH(CH_3)_2)OCH_3$, $-N(CH_3)OCH_2C_6H_5$, $-N(OCH_3)CH_2C_6H_5$, $-N(CH_3)OC_6H_5$, $-NH-CH_2-C_6H_5$, $-NH(CH_2)_2C_6H_5$, $-NH(CH_2)_3C_6H_5$, $-NHCH(CH_3)C_6H_5$, $-NHC(CH_3)_2C_6H_5$, $-NHC(CH_3)_2CH_2CH_3$, $-NHC(CH_3)(CH_2CH_3)_2$, $-NHCH(CH_3)CH(OH)C_6H_5$, $-NHCH_2$-cyclohexyle, $-NH-CH_2CF_3$, $-NHCH(CH_2F)_2$, $-NHC(CH_3)_2CH_2CH_2OH$, $-NH(CH_2CH_2O)_2CH_2CH_3$, $-NHC(CH_3)_2CH(CH_3)_2$, $-NHC(CH_3)_2CN$, $-NHC(CH_3)_2CCH$, noréphédryle, norpseudoéphédryle, -NH-quinoléyle, -NH-pyrazyle, -NH-adamantyle(2), -NH-adamantyle(1), $-NH-CH_2$-adamantyle, $-NH-CH_2$-naphtyle, -NH-benzhydryle, -NH-biphényle, -NH-pyridyle, $-NH-CH_2$-pyridyle, $-NH-CH_2-CH_2$-pyridyle, -NH-benzothiazolyle, -NH-benzoisothiazolyle, -NH-benzopyrazolyle, -NH-benzoxazolyle, -NH-fluorényle, -NH-pyrimidyle, $-NH-CH_2$-(4-méthyl)thiazolyle(2), $-NH-CH_2$-furanyle(2), $-NH-CH_2$-thiényle(2), $-NH-CH_2$-(5-méthyl)thiényle(2), -NH-thiazolyle(2), -NH-isoxazolyle(3), -NH-(3-méthyl)isoxazolyle(5), -NH-(3-méthyl)isothiazolyle(5), -NH-(2-trifluorométhyl)thiadiazolyle(5), -NH-(2-cyclopropyl)thiadiazolyle(5), $-NHC(CH_3)_2C{=}CH_2$; ou K peut être

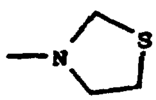
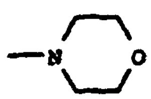
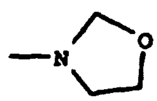
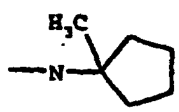
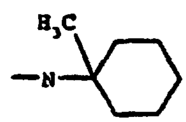

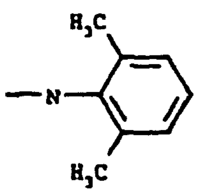
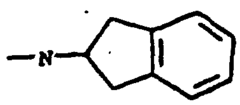
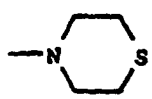
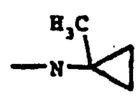
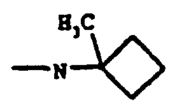

**4.** Composés selon l'une quelconque des revendications 1 à 3, pour une utilisation en médecine, en particulier pour le traitement de maladies oncologiques.

**5.** Composition pharmaceutique comprenant un support pharmaceutiquement acceptable et une quantité thérapeutiquement efficace d'un composé selon l'une quelconque des revendications 1 à 3.

**6.** Utilisation d'un composé selon l'une quelconque des revendications 1 à 3 pour la préparation d'une composition pharmaceutique destinée au traitement d'une tumeur chez un animal.

**7.** Procédé de préparation de composés selon l'une quelconque des revendications 1 à 3, **caractérisé en ce qu'**on les prépare selon des procédés connus de la chimie des peptides.

SEQUENCE LISTING

(1) GENERAL INFORMATION

(i) APPLICANT:

(A) BASF Aktiengesellschaft
(B) STREET: Carl-Bosch-Strasse 38
(C) CITY: Ludwigshafen
(E) COUNTRY: Bundesrepublik Deutschland
(F) ZIP: D-67056
(G) TELEPHONE: 0621/6048526
(H) TELEFAX: 0621/6043123
(I) TELEX: 1762175170

(ii) TITLE OF INVENTION: Novel peptides, the preparation and use thereof

(iii)NUMBER OF SEQUENCES: 4

(iv) COMPUTER READABLE FORM:

(A) MEDIUM TYPE: Diskette, 3,5 inch, 2 DD
(B) COMPUTER: IBM AT-compatible, 80286 processor
(C) OPERATING SYSTEM: MS-DOS version 5.0
(D) SOFTWARE: WordPerfect

(2) INFORMATION FOR SEQ ID NO: 1:

(i) SEQUENCE CHARACTERISTICS:

(A) LENGTH: 5 amino acids
(B) TYPE: amino acid
(D) TOPOLOGY: linear

(ii) MOLECULE TYPE: peptide
(xi) SEQUENCE DESCRIPTION: SEQ ID NO: 1:

**Xaa Val Xaa Xaa Xaa**

(2) INFORMATION FOR SEQ ID NO: 2:

(i) SEQUENCE CHARACTERISTICS:

(A) LENGTH: 5 amino acids
(B) TYPE: amino acid
(D) TOPOLOGY: linear

(ii) MOLECULE TYPE: peptide
(xi) SEQUENCE DESCRIPTION: SEQ ID NO: 2:

**Xaa Ile Xaa Xaa Xaa**

(2) INFORMATION FOR SEQ ID NO: 3:

(i) SEQUENCE CEARACTERISTICS

(A) LENGTH: 5 amino acids
(B) TYPE: amino acid
(D) TOPOLOGY: linear

(ii) MOLECULE TYPE: peptide
(xi) SEQUENCE DESCRIPTION: SEQ ID NO: 3:

**Xaa Xaa Xaa Xaa Xaa**

(2) INFORMATION FOR SEQ ID NO: 4:

(i) SEQUENCE CHARACTERISTICS:

(A) LENGTH: 6 amino acids
(B) TYPE: amino acid
(D) TOPOLOGY: linear

(ii) MOLECULE TYPE: peptide
(xi) SEQUENCE DESCRIPTION: SEQ ID NO: 4:

Xaa Val Xaa Xaa Pro Xaa